# EUROPEAN PATENT SPECIFICATION

(11) **EP 2 307 563 B1**
(45) Date of publication and mention of the grant of the patent: **06.08.2014**
(21) Application number: 09724342.2
(22) Date of filing: 30.03.2009
(51) Int. Cl.: C12Q 1/68

(54) **MUCOSAL GENE SIGNATURES**
MUCOSA-GENSIGNATUREN
SIGNATURES GÉNÉTIQUES DES MUQUEUSES

(30) Priority: 28.03.2008 US 72200 P
(43) Date of publication of application: 13.04.2011
(73) Proprietor: Katholieke Universiteit Leuven KU Leuven Research & Development, 3000 Leuven (BE)
(72) Inventor: RUTGEERTS, Paul, B-3052 Blanden (BE); SCHUIT, Frans, B-3140 Keerbergen (BE); VERMEIRE, Séverine, 3000 Leuven (BE)
(74) Representative: Laenen, Bart Roger Albert
(86) International application number: PCT/BE2009/000021
(87) International publication number: WO 2009/117791

(56) References cited:
- EP-A1- 1 172 444
- WO-A1-2004/107240
- WO-A2-2007/135568
- WO-A2-2008/028044
- CHARLES P ET AL: "Regulation of cytokines, cytokine inhibitors, and acute-phase proteins following anti-TNF-alpha therapy in rheumatoid arthritis.", JOURNAL OF IMMUNOLOGY (BALTIMORE, MD. : 1950) 1 AUG 1999 LNKD- PUBMED:10415055, vol. 163, no. 3, 1 August 1999 (1999-08-01), pages 1521-1528, XP002620410, ISSN: 0022-1767
- ARIJS I ET AL: "Mucosal gene signatures to predict response to infliximab in patients with ulcerative colitis", GUT, vol. 58, no. 12, December 2009 (2009-12), pages 1612-1619, XP002620412, ISSN: 0017-5749

## Description

### Background and Summary

### BACKGROUND OF THE INVENTION

### A. Field of the Invention

The present invention concerns mucosal gene signatures to predict response to an anti TNFα therapy such as for instance infliximab in patients with inflammatory bowel disease (IBD).

Ulcerative colitis (UC) is a chronic inflammatory bowel disease involving the mucosa of the colon distal to the anal verge. The pathogenesis of UC is believed to be a result of an interaction of genetic factors, the immune response to microbial dysbiosis and environmental factors. Cigarette smoking and appendectomy have been both associated with a decreased risk of developing UC (1). Five-aminosalicylates, corticosteroids and azathiopurine are the current treatments for UC and patients who fail these treatments were until recently referred for colectomy. The ACT trials have shown that infliximab (Remicade; Centocor, Inc., Malvern, PA, USA), a mouse/human chimeric monoclonal IgG1 antibody to tumour necrosis factor alpha (TNF-alpha), is efficacious in the treatment of patients with refractory UC and may avoid colectomy(2). However, around 40% of patients treated do not respond to infliximab and predictors of response are currently lacking.

Microarray technology is a powerful tool that enables the measurement of the expression of thousands of genes simultaneously (3). This technology has been used to elucidate the pathogenic processes underlying different diseases and to identify predictive gene profiles (4;5). An initial aim of the present study was to identify mucosal gene signatures predictive of response to anti TNFα therapeutic antibodies such for instance infliximab, adalimumab or etanercept in anti-TNF-alpha naive UC patients using high-density oligonucleotide arrays. We studied two independent cohorts of patients, one at the University Hospital Leuven (cohort A) and one cohort of patients who took part in the placebo-controlled ACT1 study(2) (cohort B). Infliximab (IFX) is such anti TNFα therapeutic antibodies that is an effective treatment for Crohn's disease (CD) and ulcerative colitis (UC) not responding to standard therapy. 30-40% of patients do not improve and response is often incomplete. Since the mechanism of resistance to anti-TNF-alpha is unknown, the aim of the study leading to the present invention was to identify mucosal gene signatures predictive of response to IFX using high-density oligonucleotide arrays. This study used colonic mucosal gene expression to provide a predictive response signature for infliximab treatment in IBD, where under UC and CD.

Gene array studies of UC mucosal biopsies identified predictive panels of genes for (non-) response to an anti-TNFalpha treatment of inflammatory bowel disease (IBD) such as Crohn's disease (CD) or ulcerative colitis (UC), or for use in monitoring the effectiveness of therapy of inflammatory bowel disease (IBD) such as Crohn's disease (CD) or ulcerative colitis (UC) in patients. This is particular suitable for predicting the effectiveness of therapy with anti TNFα therapeutic antibodies such for instance infliximab, adalimumab or etanercept. Moreover our gene array studies of mucosal biopsies identified IL-13Ralpha2 in IBD as a predictor of (non-)response to a therapy of blocking tumour necrosis factor alpha (TNFα) for instance to IFX.

WO2008028044 discloses multiple differentially expressed genes as markers for ulcerative colitis treatment with infliximab. One of said markers being IL-13Rα2, further markers associated with successful treatment against UC include CSF-receptors, NCF2, TLR2, TREM1, IL23A and IL8-Rβ.

### SUMMARY OF THE INVENTION

Crohn's disease (CD) and ulcerative colitis (UC) are complex and heterogeneous inflammatory bowel diseases (IBD), resulting from an interplay of immune, genetic and environmental factors. New insights into the pathogenesis of IBD have led to the development of biological therapies. Infliximab (Remicade; Centocor Inc., Malvern, PA, USA), a mouse/human chimeric monoclonal IgG1 antibody to tumour necrosis factor alpha, was the first clinically available biological therapy for IBD. Infliximab (IFX) is now used for over 7 years for the treatment of refractory luminal and fistulizing CD(1-4). The ACT studies have shown that IFX is also efficacious for inducing and maintaining clinical remission and mucosal healing in patients with moderate to severe, active UC who had an inadequate response to standard therapy(5). Up to 30% of patients with CD or UC do not respond to this treatment and in 20-30% response is incomplete. IFX is a costly therapy and may be associated with serious side effects. Therefore it is of critical importance to identify predictors of response to IFX.

Forty-three patients with inflammatory bowel disease (IBD), 19 with Crohn's colitis and 24 with UC, underwent a colonoscopy with biopsies before and 4 to 6 weeks after the first IFX treatment. Response to IFX was defined as endoscopic and histologic healing. Total RNA was isolated from pre-IFX biopsies, labelled and hybridized to Affymetrix HGU133plus2.0 Array. Micorarray data were analyzed using Bioconductor software. Quantitative real time RT-PCR and immunohistochemistry were used to confirm microarray data. Furthermore two cohorts of patients who received their first treatment with infliximab for refractory UC were studied. Response to infliximab was defined as endoscopic and histologic healing. Total RNA was isolated from colonic mucosal biopsies, labelled and hybridized to Affymetrix Human Genome U133 Plus 2.0 Arrays. Data were analyzed using Bioconductor software. Quantitative RT-PCR was used to confirm microarray data. Eight UC patients and 12 CD patients showed healing. In UC, only one probe set was differentially expressed in responders compared with non-responders, i.e. IL-13Ralpha2. At PAM analysis 2 probe sets, representing IL-13Ralpha2 and IL-11, separated IBD responders from non-responders with an overall misclassification error rate of 0.046 (2/43), with 100% sensitivity and 91.3 % specificity. The IL-13Ralpha2 probe set was a top-ranked probe set in all our analyses using both LIMMA and PAM strategies. For predicting response to infliximab treatment, pre-treatment colonic mucosal expression profiles were compared for responders and non-responders. Comparative analysis identified 179 significant probe sets in cohort A and 361 in cohort B with an overlap of 74 probe sets, representing 53 known genes, between both analyses. Comparative analysis of both cohorts combined yielded 212 significant probe sets. The top 5 significant genes in a combined analysis of both cohorts were osteoprotegerin, stanniocalcin-1, prostaglandin-endoperoxide synthase 2, interleukin-13 receptor alpha 2 and interleukin-11. All proteins encoded by these genes are involved in the adaptive immune response. These markers separated responders from non-responders with 95% sensitivity and 85% specificity.

The present invention is based on the surprising finding that increased IL-13Ralpha2 expression or IL-13Ralpha2 activity is suppressive on the response to an anti TNFα therapy of inflammatory bowel disease (IBD)such as for instance infliximab in patients with inflammatory bowel disease (IBD). This finding indicated that the non responding to such anti TNFα therapy of IBD, in particular UC or CD can be attenuated by inhibiting the IL-13Ralpha2 expression or activity. Such interventions have been proposed as a pharmaceutical co treatment with anti TNFα therapy of inflammatory bowel disease by the present invention.

### Detailed Description

### DETAILED DESCRIPTION OF EMBODIMENTS OF THE INVENTION

Unless defined otherwise, all technical and scientific terms used herein have the same meaning as is commonly understood by one of ordinary skill in the art to which this invention belongs. All patents, applications, published applications and other publications are incorporated by reference in their entirety. In the event that there are a plurality of definitions for a term herein, those in this section prevail unless stated otherwise.

Abbreviations in the applications are FC, fold change; FDR, false discovery rate; IL-11, interleukin-11; IL-13Ralpha2, interleukin-13 receptor alpha 2; IQR, interquartile range; LIMMA, linear models for microarray data; NR, non-responders; PAM, prediction analysis of microarrays; PTGS2, prostaglandin-endoperoxide synthase 2; qPCR, quantitative RT-PCR; R, responders; RMA, robust multichip average; STC1, stanniocalcin-1; TNF-alpha, tumour necrosis factor-alpha; TNFRSF11B, osteoprotegerin; UC: ulcerative colitis Homo sapiens interleukin 13 receptor, alpha 2 (IL13RA2) has been described in Katsoulotos,G.P., et al. J. Biol. Chem. 283 (3), 1610-1621 (2008); Tanabe,Tet al. Clin. Exp. Allergy 38 (1), 122-134 (2008); Bozinov,O., et al; Neurosurg Rev 31 (1), 83-89 (2008); Jarboe,J.S., et al. Cancer Res. 67 (17), 7983-7986 (2007); Andrews,A.L. el J. Allergy Clin. Immunol. 120 (1), 91-97 (2007); Donaldson,D.D., et al. J. Immunol. 161 (5), 2317-2324 (1998); Guo,J., et al. Chromosome mapping and expression of the human interleukin-13 Genomics 42 (1), 141-145 (1997); Zhang,J.G., et al; J. Biol. Chem. 272 (14), 9474-9480 (1997); Gauchat,J.F., et al; Eur. J. Immunol. 27 (4), 971-978 (1997) and Caput,D., et al. J. Biol. Chem. 271 (28), 16921-16926 (1996) and a sequence for CDR and mRNA has for instance been deposited at NCBI under accession NM_000640 (PUBMED 8663118 LOCUS NM_000640 1376 bp mRNA linear PRI 17-FEB-2008).

Infliximab is a therapeutic monoclonal antibody that Infliximab works by blocking tumour necrosis factor alpha (TNFα, a chemical messenger (cytokine) which a key part of the autoimmune reaction. Infliximab blocks the action of TNFα by preventing it from binding to its receptor in the cell. Other anti TNFα therapeutic antibodies are for instance Adalimumab (brand name HUMIRA) or etanercept. Like infliximab, etanercept and adalimumab binds to TNFα, preventing it from activating TNF receptors. The present invention provides a diagnostic method to predict response or non response in an inflammatory bowel disease (IBD) and more particularly ulcerative colitis treatment by compounds that block the action of TNFα such as Infliximab, Adalimumab or Etanercept

Forty-three patients with active IBD, 24 UC and 19 CD, refractory to corticosteroids and/or immunosuppression and a control group of 6 individuals who underwent colonoscopy for screening were studied. Baseline characteristics of the patients are summarized in table 1. The patients underwent colonoscopy with biopsies from diseased colon within a week prior to the first intravenous infusion of 5 mg IFX per kg body weight. The patients underwent a second colonoscopy 4 weeks after the first IFX infusion in case of a single infusion and at 6 weeks if they received a loading dose of IFX at weeks 0, 2 and 6. Part of the biopsies was immediately snap-frozen in liquid nitrogen and stored at -80°C until RNA isolation. The residual biopsies were fixed in Carnoy's fixative for up to 5 hours and then dehydrated, cleared and paraffin-embedded for histologic examination.
All patients were followed-up long-term. The ethics committee of the university hospital approved the study and all individuals gave written informed consent.
Reponse to IFX was defined as a complete mucosal healing with a decrease of at least 3 points on the histological score for CD(9) and as a decrease to a Mayo endoscopic subscore of 0 or 1(5) with a decrease to grade 0 or 1 on the histological score for UC(10). Patients who did not achieve this healing were considered non-responders although some of them presented improvement.

### EXAMPLES

### Example 1

### RNA isolation:

Total RNA was extracted from the biopsy specimens using the RNeasy Mini Kit (Qiagen, Benelux B.V.), according to the manufacturer's instructions. The integrity and quantity of total RNA were assessed with the Agilent 2100 Bioanalyzer (Agilent, Waldbronn, Germany) and Nanodrop ND-1000 spectrophotometer (Nanodrop technologies). The extracted RNA was used for micorarray analysis and in some cases for quantitative RT-PCR analysis.

### Oligonucleotide array hybridization:

All steps were performed according to Affymetrix expression analysis technical manual 701021Rev.5 (Santa Clara, CA, USA). Briefly, total RNA (2 µg) was reverse-transcribed into cDNA using the SuperScript Choice System (Invitrogen, Carlsbad, CA, USA). cDNA was *in* vitro transcribed to cRNA and biotin labeled (Affymetrix, Santa Clara, CA, USA). Biotinylated cRNA was purified and fragmented. The quality of labeled and fragmented cRNA, respectively, was assessed with the Agilent 2100 Bioanalyzer. Fragmented cRNA (15 µg) was hybridized overnight to the Human Genome U133 Plus 2.0 Array (Affymetrix, Santa Clara, CA, USA). The arrays were washed and stained with streptavidin-phycoerytrin and scanned on the Affymetrix 3000 GeneScanner. The resulting image files (.dat files) were analyzed using Affymetrix GCOS software, and intensity values for each probe cell (.cel file) were calculated. Quality evaluation of the microarrays were as expected. The data are available at ArrayExpress, a public repository for microarray data (accession number and address).

### Data analysis

The affymetrix raw data (.cel files) were analyzed using Bioconductor tools(11) in R (version 2.4.1, http://www.r-project.org/). Probe level analysis was performed with the robust multichip average (RMA) method(12). Linear models for microarray data (LIMMA)(13) and prediction analysis of microarrays (PAM)(14) were used for supervised data analyses. LIMMA was used to identify differentially expressed (DE) probe sets between the groups, on the basis of moderated t-statistics using an empirical Bayes method, with the use of false discovery rate (FDR) correction for multiple testing (Benjamini and Hochberg(15)). A greater than 2-fold change combined with a FDR < 0.05 were considered statistically significant. PAM, a nearest shrunken centroid method, was used to identify a subset of probe sets which can be used to classify pre-treatment samples as responder (R) or non-responder (NR). PAM was applied to the entire dataset using leave-one-out cross validation. Unsupervised hierarchical clustering based on the average-linkage method with the Euclidian distance metric was performed to visualize gene (probe set)/sample relationship. The Functional Annotation tool on the DAVID homepage (http://david.abcc.ncifcrf.gov/home.jsp) was used for Gene Ontology (GO) analysis to find biological processes that are overrepresented among sets of DE probe sets(16). Only GO categories represented by more than 10 probe sets in the set of DE probe sets and an EASE score < 0.01 were considered significant.

### Quantitative RT-PCR

To validate the microarray data, quantitative duplex real time RT-PCR for the genes IL-13Ralpha2 and β-actin (the endogenous reference gene) was performed, using total RNA from samples that were used for the microarrays. cDNA was synthesized from 0.5 µg of total RNA using the RevertAid H Minus First Strand cDNAsynthesis kit (Fermentas, St. Leon-Rot, Germany), following the manufacturer's protocol. Primers and dual-labeled probes were designed using OligoAnalyzer 3.0 software (http://biotools.idtdna.com/analyzer/) and synthesized by Sigma-Genosys (Haverhill, UK). The oligonucleotide sequences are available upon request. Real-time PCR was performed in a final reaction volume of 25 µl on a Rotor-Gene 3000 instrument (Corbett Research, Mortlake, Australia), using QuantiTect Multiplex PCR NoROX Kit (Qiagen, Venlo, NL), according to the manufacturer's instructions. Cycle threshold values were determined by Rotor-Gene 6.0.16 software. All samples were amplified in duplicate reactions. The relative expression of target mRNA levels were calculated as a ratio relative to the β-actin reference mRNA(17). Results were analyzed using the Mann-Whitney *U*-test using SPSS 15.0 software (SPSS, Chicago, IL) and a *P*-value of < 0.05 was considered significant.

### IL-13Ralpha2 immunohistochemistry

To determine the IL-13Ralpha2 protein localization, immunohistochemical staining was performed on 5 µm-thick step sections prepared from each paraffin block. Endogenous peroxidase activity was blocked in dewaxed sections by incubating the slides for 20 minutes in a 0.3% solution of H₂O₂ in methanol. Epitope retrieval was performed by heating the slides for 30 minutes in Tris/EDTA buffer (pH 9) at 98°C. Sections were then incubated with the antihuman IL-13Ralpha2 mouse monoclonal antibody clone ab55275 (Abcam plc, Cambridge, United Kingdom) at a concentration of 1 µg/mL for 30 minutes. The Dako REAL^{™} Envision^{™} Detection System kit (Dako Belgium NV, Heverlee, Belgium) was used for visualization of bound primary antibody according to the manufacturer's instructions. Formalin-fixed, paraffin-embedded surgical biopsies of an ovarian serous adenocarcinoma served as positive controls(18). The primary antibody was omitted in the negative controls.

### Study of the IL-13, IL-13Ralpha1, IL-13Ralpha2 and TGF-beta1 pathway

In order to identify mechanisms of non-response, we compared the gene intensities for pathway related molecules including IL-13, IL-13Ralpha1, IL-13Ralpha2 and TGF-beta1 in R and NR and explored the relationships between the probe sets. The correlation between probe sets were analyzed using the Spearman's Rank Correlation test using SPSS 15.0 software.

### Results:

### Identification of differentially expressed probe sets between R and NR:

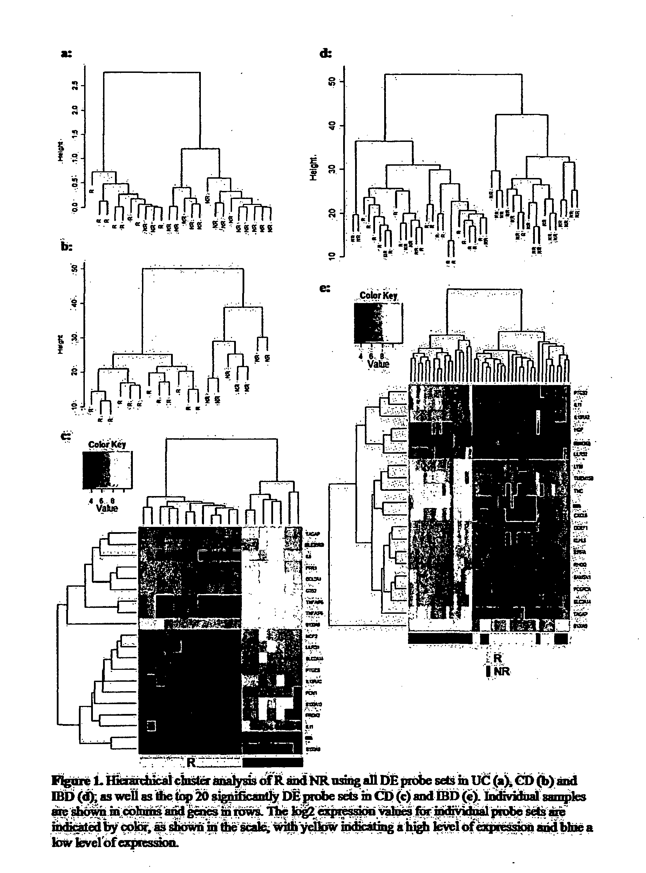

We identified 20 R (8 UC and 12 CD) and 23 NR (16 UC and 7 CD). There were no baseline characteristics predictive of response. For predicting response to IFX treatment based on gene profiles, pre-treatment expression profiles were compared for R and NR, using LIMMA. In UC, only 1 probe set, representing IL-13Ralpha2, was DE between R and NR with an upregulation in NR. Hierarchical clustering, based on the log₂ expression values of IL-13alpha2 from UC patients, resulted in 2 major clusters of R versus NR, with two NR misclassified in the cluster of R (Figure 1a). At LIMMA analysis a total of 697 probe sets were DE in CD, with 5 probe sets showing an increased expression and 692 a decreased expression in R compared with NR. Hierarchical cluster analysis using all DE probe sets (Figure 1b), as well as the top 20 significantly DE probe sets (Figure 1c) completely separated R from NR in CD. For IBD overall (UC and CD), 672 DE probe sets were identified between R and NR. Thirty-one of the 672 DE probe sets showed an increased expression and 641 probe sets showed a decreased expression in R compared to NR. Cluster analysis, using all DE probe sets (Figure 1d), as well as the top 20 significantly DE probe sets (Figure 1e), showed 2 major clusters of R and NR for IBD overall, with 8/43 and 6/43 misclassifications, respectively. The DE probe sets from each pairwise comparison are listed in Supplementary table 1. To identify which biological processes are associated with (non-)response to IFX treatment, we performed a GO analysis on the DE probe sets from the pairwise comparisons between R and NR in CD and IBD (Supplementary table 2). The DE probe sets included genes that were predominantly involved in immune response, inflammatory response, signal transduction, cell adhesion, response to wounding, response to pest, pathogen or parasite, apoptosis, chemotaxis.

### Class prediction analysis of R and NR:

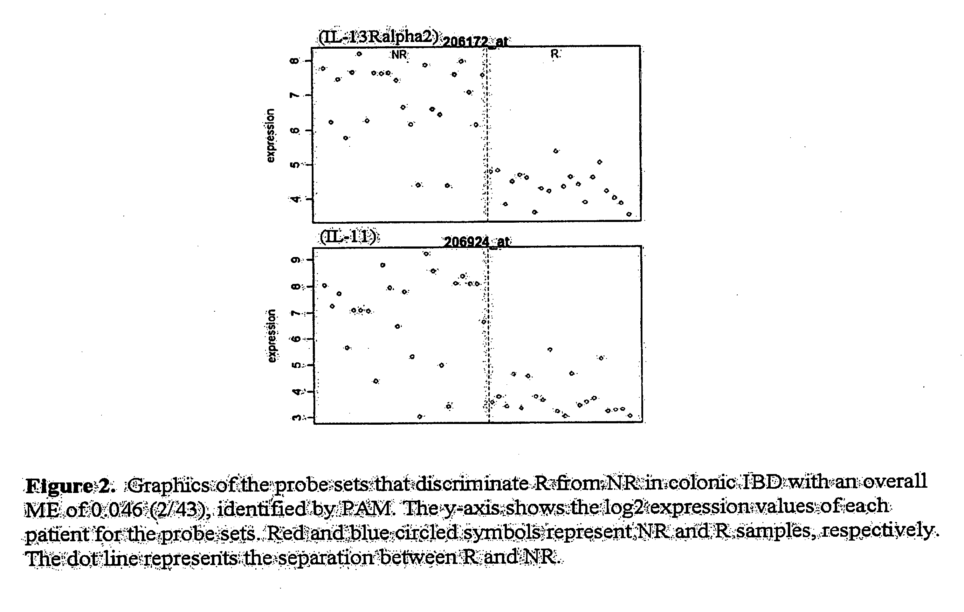

PAM was carried out on the pre-treatment expression profiles from R and NR to identify the smallest subset of probe sets that were able to accurately predict response or non-response with the lowest misclassification error (ME) rate. A subset of 37 probe sets was necessary in UC to classify samples as R or NR to achieve an overall ME rate of 0.165 (4/24) and 21 probe sets were necessary in CD to achieve a ME rate of 0 (0/15). Two probe sets, representing IL-13Ralpha2 and IL-11, allowed nearly complete separation between R and NR in IBD overall with a ME rate of 0.046 (2/43), classifying all R (sensitivity 100%) and 21 of 23 NR (specificity 91.3%) correctly (Figure 2). The subsets of probe sets, identified by PAM analysis in UC, CD and IBD are listed in Supplementary table 3.

### IL-13Ralpha2:

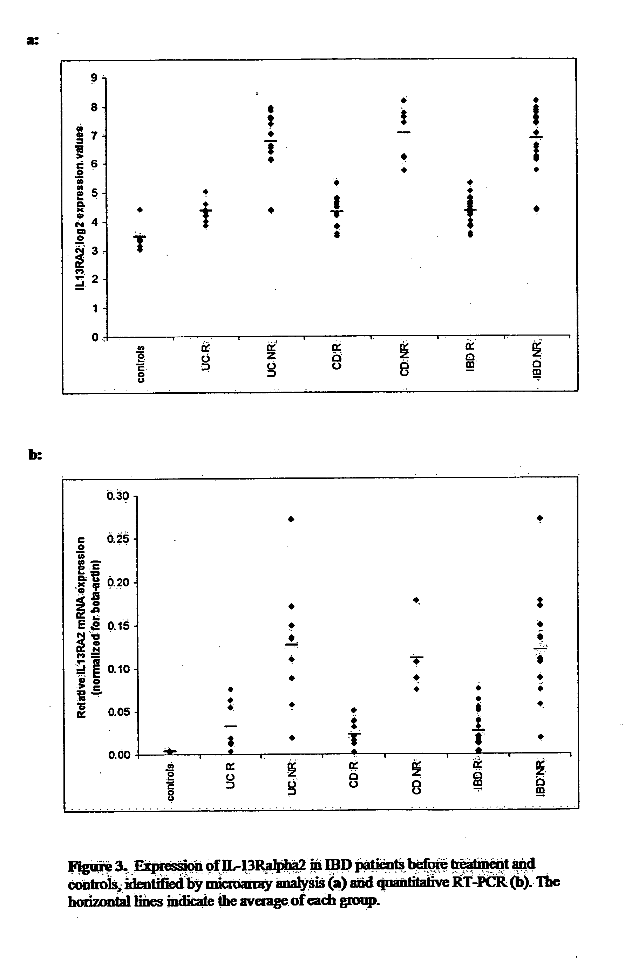

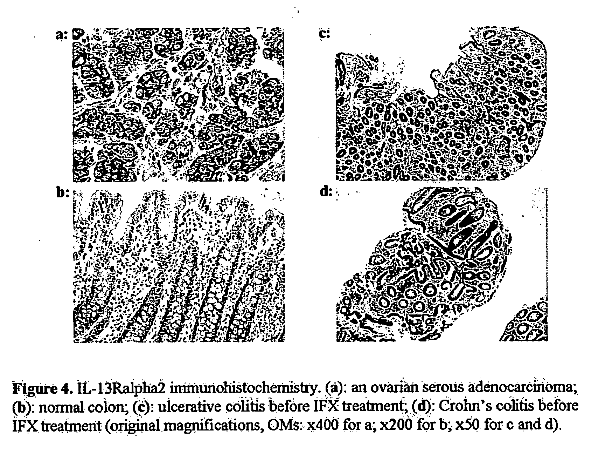

The probe set, representing IL-13Ralpha2, was of special interest because it was present as a top-ranked probe set for both the LIMMA and PAM analyses. LIMMA analysis showed also significantly increased mRNA expression of IL-13Ralpha2 in both untreated UC and CD compared to controls (Figure 3a). Quantitative RT-PCR of IL-13Ralpha2 confirmed the differential expression of IL-13Ralpha2 between the different groups (R, NR and controls) (Figure 3b). To determine where IL-13Ralpha2 is expressed in the colonic mucosa, immunohistochemistry was performed. The staining for IL-13Ralpha2 performed as expected in the positive and negative controls. In the normal colon, IL-13Ralpha2 was located mainly in the cytoplasm of the goblet cells. Compared with normal colon, CD and UC biopsies showed an increased staining intensity in all epithelial cells. There was no restriction to goblet cells. There was also no difference in staining intensity between CD and UC (Figure 4).

### The IL-13, IL-13Ralpha1, IL-13Ralpha2 and TGF-beta1 pathway

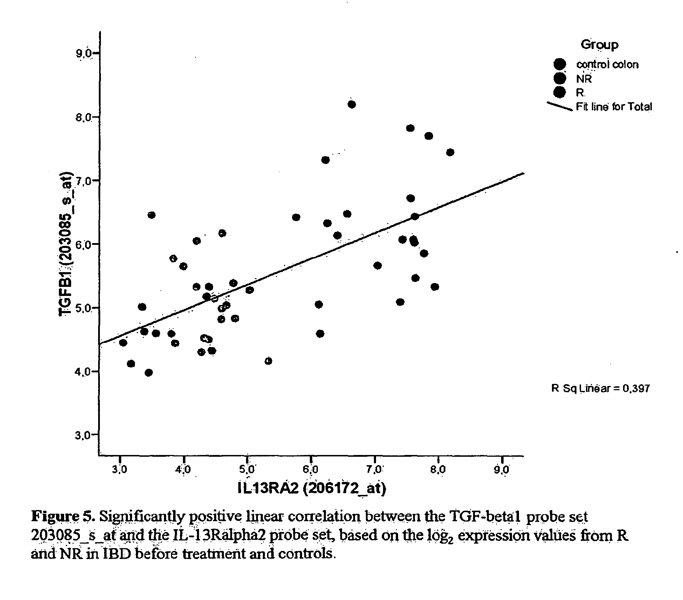

Probe sets for IL-13 and IL-13Ralpha1 were not DE between R and NR and were not upregulated in comparison with controls. The TGF-beta1 probe set 203085_s_at and the IL-13Ralpha2 probe set showed a significantly (P-value_{Spearman rank correlation}<0.001) positive linear correlation, based on the log₂ expression values of IBD R and IBD NR before treatment and controls (Figure 5).

### Discussion

There is a great need to identify predictors of (non-)response to anti-TNF therapies and to biological therapies in general. We investigated whether mucosal gene signatures predictive of response to infliximab can be identified. We studied patients with refractory IBD who had never been treated with biological therapy.
Since the reported efficacies of infliximab in Crohn's disease and ulcerative colitis are very similar our hypothesis was that the predictors would be the same for both diseases.
Using stringent criteria of response, namely endoscopic and histologic healing we identified one single probe set IL-13Ralpha2 which was DE at LIMMA when comparing R and NR in UC. In Crohn's disease and IBD overall many more probe sets were DE but IL-13Ralpha2 was always one of the top-ranked probes. At PAM analysis 2 probe sets, representing IL-13Ralpha2 and IL-11, allowed separation of R and NR in IBD overall with a ME rate of 0.046 and a sensitivity 100% and specificity of 91.3%.
In contrast with IL-11 , IL-13Ralpha2 was a top-ranked probe set in all analyses. This receptor has raised a lot of interest lately as this receptor is involved in fibrogenesis. IL-13 is a critical regulator of a Th2 response and is the key cytokine in parasyte immunity and in the generation of an allergic response. Two members of the type 5 subfamily of type I cytokine receptors can serve as receptors for IL-13. IL-13 can bind to IL-13Ralpha1 with low affinity, then recruits the IL-4Ralpha chain to form a high affinity receptor, causing downstream STAT6 activation.
Alternately, IL-13 together with TNF can induce IL-13Ralpha2 (CD213a2) with high affinity. Interaction between IL-13 and IL-13Ralpha2 does not activate STAT6, and originally it was believed that IL-13Ralpha2 acts as a decoy receptor. Recently it was shown however that the interaction leads to activation of the TGF-Beta1 promoter(19).
In the model of chronic TNBS colitis mimicking IBD an initial Th1 response subsides after 3 weeks to be supplanted by an IL-23/IL-25 response beginning after 4-5 weeks. This evolution is followed by gradually increasing production of IL-17 and cytokines ordinarily seen in a Th2 response, particularly IL-13, which reaches a plateau at 8-9 weeks. IL-13 production results in the induction of IL-13Ralpha2, inducing TGF-beta1 and the onset of fibrosis(20).
If IL-13 signalling through this receptor is blocked by administration of soluble IL-13Ralpha2-Fc, or by administration of IL-13Ralpha2-specific small interfering RNA, TGF-beta1 is not produced and fibrosis does not occur.
Our study shows that in UC and probably also in a subset of CD upregulation of IL-13Ralpha2 is responsible for resistance to anti-TNF therapy. The effect is probably due to the induction of TGF-beta1 as we found a good correlation between expression of IL-13Ralpha2 and TGF-beta1. Our study would suggest that IL-13Ralpha2 expression is a good predictor of response to anti-TNF and that patients not responding to anti-TNF are candidates to receive blockade of the IL-13/IL-13Ralpha 2 pathway or of TGF-beta1. Alternatively patients with high RT-PCR levels of IL-13Ralpha2 might receive combined blockade of TNF and TGF-beta1 from the onset.

We identified mucosal IL-13Ralpha2 as a predictor for (non-)response to anti-TNF therapy in IBD and in particular of UC.

### Example 2

### Patients

### Cohort A

Twenty-four patients with active UC, refractory to corticosteroids and/or immunosuppression, were studied in cohort A. The study was carried out at the University Hospital of Gasthuisberg in Leuven (tertiary referral center) (ClinicalTrials.gov number, NCT00639821) and all patients were followed-up long-term. The ethics committee of the University Hospital approved the study and all individuals gave written informed consent.
The baseline characteristics of the UC patients from cohort A are summarized in table 1a.

**Table 1a Baseline characteristics of the UC patients from cohort A**

| | **Responders(n=8)** | **Non-responders (n=16)** |
|---|---|---|
| Male/Fernal (%) | 4/4 (50/50) | 10/6 (62.5/37.5) |
| Median (IQR) age at first IFX (years) | 28.4 (24.3-41.8) | 45.8 (36.5-62.3) |
| Median (IQR) weight at first IFX (kg) | 72 (57.8-78.5) | 73.3 (68.5-80.3) |
| Median (IQR) duration of disease prior to first IFX (years) | 10.3 (4.1-17.3) | 7.3 (2.6-13.3) |
| Median (IQR) C-reactive protein at first IFX (mg/dL) | 1.65 (1-9.6) | 6.5 (2.9-19.1) |
| Concomitant medication at first IFX (%) | | |
| 5-Aminosalicylates | 5 (62.5) | 13 (81.3) |
| Corticosteroids | 2 (25) | 5 (31.3) |
| Azathioprine/6-Mercaptopurine | 7 (87.5) | 8 (50) |
| Corticosteroids + Iminunosuppressants | 2 (25) | 1 (6.3) |
| Active smoking at first IFX (%) | 1 (12.5) | 1 (6.3) |

| | | |
|---|---|---|
| IQR, interquatile range; IFX, infliximab | | |

In the study consecutive patients with active UC who consented with the study were included. The patients had a total Mayo score between 6-12 and an endoscopic subscore of at least 2(Rutgeerts P, Sandborn WJ, Feagan BG et al. Infliximab for induction and maintenance therapy for ulcerative colitis. N Engl J Med 2005;353(23):2462-76). The UC patients underwent colonoscopy with biopsies from diseased rectum within a week prior to the first intravenous infusion of 5 mg infliximab per kg body weight. The patients underwent a second flex sigmoidoscopy with rectal biopsies 4 weeks after the first infliximab infusion in case of a single infusion and at 6 weeks if they received a loading dose of infliximab at weeks 0, 2 and 6. The endoscopist was not blinded to treatment. Half of the biopsies were immediately snap-frozen in liquid nitrogen and stored at -80°C until RNA isolation. The residual biopsies were fixed in Carnoy's fixative for up to 5 hours and then dehydrated, cleared and paraffin-embedded for histologic examination. The features of chronic intestinal inflammation were scored in haematoxylin-eosin stained slides from the paraffin blocks of each patient using a previously reported scoring system for UC (Geboes K, Riddell R, Ost A et al. A reproducible grading scale for histological assessment of inflammation in ulcerative colitis. Gut 2000;47(3):404-9). The pathologists who scored the biopsies (KG and GDH) were blinded to treatment.
The response to infliximab was assessed at 4-6 weeks after first infliximab treatment and defined as a complete mucosal healing with a Mayo endoscopic subscore of 0 or 1(Rutgeerts P, Sandborn WJ, Feagan BG et al. Infliximab for induction and maintenance therapy for ulcerative colitis. N Engl J Med 2005;353(23):2462-76) and a grade 0 or 1 on the histological score for UC (Geboes K, Riddell R, Ost A et al. A reproducible grading scale for histological assessment of inflammation in ulcerative colitis. Gut 2000;47(3):404-9). Patients who did not achieve this healing were considered non-responders although some of them presented endoscopic and/or histologic improvement.
For validation of the microarray results with quantitative RT-PCR (qPCR), colonic biopsies were obtained from 6 control subjects who underwent colonoscopy for screening for polyps. These patients gave informed consent for the study.

### Cohort B

Endoscopies were carried out and biopsies were collected during the ACT1 trial(Rutgeerts P, Sandborn WJ, Feagan BG et al. Infliximab for induction and maintenance therapy for ulcerative colitis. N Engl J Med 2005;353(23):2462-76), a placebo controlled trial of infliximab therapy in refractory UC (ClinicalTrials.gov number, NCT00036439), at protocol-specified time points from a subset of randomized patients. Endoscopists and pathologists in this study were blinded to therapy. The institutional review board or ethics committee at each site approved the ACT1 protocol and all patients provided informed consent.
For the current study, 23 pre-treatment colonic mucosal biopsies from 22 active UC patients (2 colonic mucosal biopsies were obtained within two weeks from the same patient) who received their first infusion of infliximab 5 or 10 mg/kg for refractory ulcerative colitis were analyzed in cohort B. The biopsies were collected 15 to 20 centimetres distal from the anal verge. Table 1b summarized the baseline characteristics of the UC patients from cohort B.

**Table 1b Baseline characteristics of the UC patients from cohort B**

| | **Responders (n=12)** | **Non-responders (n=10)** |
|---|---|---|
| Male/Female (%) | 6/6 (50/50) | 6/4 (60/40) |
| Median (IQR) age at first IFX (years) | 39 (29-70) | 51.5 (24-68) |
| Median (IQR) weight at first IFX (kg) | 75 (63-159) | 69 (46-102) |
| Median (IQR) duration of disease prior to first IFX (years) | 5.9 (1.6-42.1) | 5.7 (2.9-26.8) |
| Median (IQR) C-reactive protein at first IFX (mg/dL) | 0.7 (0.2-2.9) | 1.35 (0.2-6.8) |
| Concomitant medication at first IFX (%) | | |
| 5-Aminosalicylates | 1 (8.3) | 1 (10) |
| Corticosteroids | 8 (66.7) | 6 (60) |
| Azathioprine/6-Mercaptopurine | 2 (16.7) | 3 (30) |
| Corticosteroids + Immunosuppressants | 0 (0) | 0 (0) |
| Active smoking at first IFX (%) | 1 (8.3) | 0 (0) |

| | | |
|---|---|---|
| IQR, interquatile range; IFX, infliximab | | |

The response to infliximab was assessed at 8 weeks after their first infliximab treatment and the definition of response was identical to cohort A.

### RNA isolation

For cohort A, total RNA was extracted from the biopsy specimens using the RNeasy Mini Kit (Qiagen, Benelux B.V.), according to the manufacturer's instructions. The integrity and quantity of total RNA were assessed with a 2100 Bioanalyzer (Agilent, Waldbronn, Germany) and Nanodrop ND-1000 spectrophotometer (Nanodrop technologies). The extracted RNA was used for microarray analysis and in some cases for qPCR analysis.
For cohort B, total RNA was isolated from the biopsies with RNeasy mini-kit according to the manufacturer's instructions (Qiagen, Valencia, CA). RNA quality and quantity were analyzed with a 2100 Bioanalyzer (Agilent Technologies Inc., Palo Alto, CA).

### Oligonucleotide array hybridization

For samples from both cohorts, all steps were performed according to Affymetrix expression analysis technical manual 701021Rev.5 (Affymetrix, Santa Clara, CA, USA). Briefly, total RNA (2 µg) was reverse-transcribed into cDNA using the SuperScript Choice System (Invitrogen, Carlsbad, CA, USA). cDNA was *in vitro* transcribed to cRNA and biotin labelled (Affymetrix). Biotinylated cRNA was purified and fragmented. The quality of labelled and fragmented cRNA, respectively, was assessed with the Agilent 2100 Bioanalyzer. Fragmented cRNA (15 µg) was hybridized overnight to the Human Genome U133 Plus 2.0 Array (Affymetrix), which comprised of 54675 probe sets. The arrays were washed, stained with streptavidin-phycoerytrin and scanned on the Affymetrix 3000 GeneScanner. The resulting image files (.dat files) were analyzed using Affymetrix GCOS software, and intensity values for each probe cell (.cel file) were calculated. Quality evaluations of the microarrays were as expected.

### Data analysis

A glossary of terms used in the analysis is provided in Supplementary Appendix 1.
The microarray data were analyzed using Bioconductor tools (Gentleman RC, Carey VJ, Bates DM et al. Bioconductor: open software development for computational biology and bioinformatics. Genome Biol 2004;5(10):R80) in R (version 2.7.2, http://www.r-project.org/). The robust multichip average (RMA) method (Irizarry RA, Hobbs B, Collin F et al. Exploration, normalization, and summaries of high density oligonucleotide array probe level data. Biostatistics 2003;4(2):249-64.) was performed on the affymetrix raw data (.cel files) from both cohorts to obtain an expression value for each probe set. Because the severe correction for 54675 comparisons may have caused false negatives, we next performed the analysis with probe sets that hybridized above background levels to the patient samples. The probe sets with low overall intensity and variability that are unlikely to carry information about the phenotypes under investigation were removed. A non-specific filtering was applied on the log2 RMA normalized data from the pre-treatment UC samples from both cohorts. Only probe sets with an intensity > log2(100) in at least 10% of the samples and an interquartile range (IQR) of log2 intensities across the samples > 0.5 were included, leaving 9183 probe sets for further data analysis (Supplementary table 1). Probe set annotations were obtained through the Affymetrix NetAffx website (http://www.affymetrix.com/analysis/index.affx) or the UCSC Genome Browser website (http://genome.ucsc.edu/). Linear models for microarray data (LIMMA) (Smyth GK. Linear models and empirical bayes methods for assessing differential expression in microarray experiments. Stat Appl Genet Mol Biol 2004;3:Article3) and prediction analysis of microarrays (PAM) (Tibshirani R, Hastie T, Narasimhan B et al. Diagnosis of multiple cancer types by shrunken centroids of gene expression. Proc Natl Acad Sci USA 2002;99(10):6567-72) were used for supervised data analyses. For comparative analysis, LIMMA was used to identify probe sets that are different between responders and non-responders, based on moderated t-statistics. To correct for multiple testing, the false discovery rate (FDR) was estimated from p-values derived from the moderated t-statistics using the method of Benjamini and Hochberg( 11). A greater than 2-fold change combined with a FDR < 0.05 were considered statistically significant. For class prediction, PAM with leave-one-out cross validation was carried out on the top 20 and the top 5 most significantly different known genes, identified by LIMMA analysis between responders and non-responders, to see if these subsets accurately predict response or non-response to infliximab and to identify the lowest misclassification error rate based on these subsets. Unsupervised average-linkage hierarchical clustering, using Euclidian distance as metric, was applied to data obtained from the data analysis to visualize gene/sample relationship. The results of the clustering were visualized as a 2-dimensional heatmap with 2 dendograms, one indicating the similarity between patients and the other indicating the similarity between genes. The Bio Functional Analysis tool in the Ingenuity Pathway Analysis program (Ingenuity Systems®, www.ingenuity.com) was used to identify biological functions and/or diseases that were most significant to the dataset of significant probe sets that were identified by LIMMA analysis between responders and non-responders. The genes represented by the significant probe sets that were associated with biological functions and/or diseases in the Ingenuity knowledge base were considered for the analysis. Fischer's exact test was used to calculate a p-value determining the probability that each biological function and/or disease assigned to that data set is due to chance alone. For multiple testing correction, the p-values were adjusted with the Benjamini and Hochberg (B-H) method.

### Quantitative RT-PCR

To validate the microarray data, qPCR was performed for osteoprotegerin (TNFRSF11B), stanniocalcin-1 (STC1), prostaglandin-endoperoxide synthase 2 (PTGS2), interleukin-13 receptor alpha 2 (IL-13Ralpha2), interleukin-11 (IL-11) and beta-actin. Beta-actin was used as the endogenous reference gene. Total RNA from samples of cohort A was used. cDNA was synthesized from 0.5 µg of total RNA using the RevertAid H Minus First Strand cDNA synthesis kit (Fermentas, St. Leon-Rot, Germany), following the manufacturer's protocol. Primers and dual-labelled probes were designed using OligoAnalyzer 3.0 software (http://biotools.idtdna.com/analyzer/) and synthesized by Sigma-Genosys (Haverhill, UK). The oligonucleotide sequences are available upon request. Duplex qPCR was performed in a final reaction volume of 25 µl on a Rotor-Gene 3000 instrument (Corbett Research, Mortlake, Australia), using QuantiTect Multiplex PCR NoROX Kit (Qiagen, Venlo, NL), according to the manufacturer's instructions. Cycle threshold values were determined by Rotor-Gene 6.0.16 software. All samples were amplified in duplicate reactions. The relative expression of target mRNA levels were calculated as a ratio relative to the beta-actin reference mRNA(Pfaffl MW. A new mathematical model for relative quantification in real-time RT-PCR. Nucleic Acids Res 2001;29(9):e45). Results were analyzed using the Mann-Whitney *U-*test using SPSS 16.0 software (SPSS, Chicago, IL) and a P-value of < 0.05 was considered significant.

### RESULTS

### Response to infliximab

Two independent UC cohorts (cohort A and cohort B) were studied to identify mucosal gene signatures predictive of response to infliximab in UC. The response to infliximab was defined as complete endoscopic and histologic healing. Of the 24 UC patients in cohort A, 8 patients were responders and 16 patients were non-responders while cohort B had 12 responders and 10 non-responders.

### Comparative analysis between responders and non-responders

For predicting response to infliximab treatment based on gene profiles, pre-treatment expression profiles were compared for responders and non-responders in each cohort and both cohorts combined, using LIMMA.
When all probe sets on the microarray (54675 probe sets) were included in the LIMMA analysis, the stringent correction for multiple testing resulted in only one significant probe set between responders and non-responders for UC in cohort A, namely IL-13Ralpha2. Therefore a non-specific filtering was first applied on the normalized data from the pre-treatment UC samples from both cohorts to eliminate non-relevant probe sets, leaving 9183 probe sets for further comparative analyses (Supplementary table 1).
In cohort A, LIMMA analysis identified a total of 179 probe sets that were significantly decreased in responders compared with non-responders (Table 2 and Supplementary table 2). In cohort B, a total of 361 probe sets were significantly different in responders compared to non-responders, with 38 probe sets showing an increased signal and 323 probe sets a decreased signal in responders compared to non-responders (Table 2 and Supplementary table 2). For the two cohorts combined (cohort A and cohort B), a total of 212 significant probe sets were identified by LIMMA analysis, with 5 probe sets showing an increased signal and 207 probe sets a decreased signal in responders compared to non-responders (Table 2 and Supplementary table 2).

**Table 2 Summary of the results from LIMMA analyses between the pre-treatment expression profiles of responders and non-responders in cohort A, cohort B and both cohorts combined**

| **Comparative analysis** | **UC cohort A R(n=8)/NR(n=16)** | **UC cohort B R(n=12)/NR(n=10)** | **UC cohort A and B R(n=20)/NR(n=26)** |
|---|---|---|---|
| Increased probe sets in R | 0 | 38 | 5 |
| Decreased probe sets in R | 179 | 323 | 207 |
| Total | 179 | 361 | 212 |

| | | | |
|---|---|---|---|
| NR, non-responders, R, responders | | | |

There was an overlap of 74 significant probe sets, representing 53 different known genes, between the LIMMA analyses in cohort A and in cohort B, and these common probe sets were all down regulated in responders as compared to non-responders (Table 3).

**Table 3 Fold change of the 74 common significant probe sets between LIMMA analysis in cohort A and LIMMA analysis in cohort B**

| **Probe Set ID*** | **Gene Symbol** | **Cohort A FC (R/NR)** | **Cohort B FC(R/NR)** | **Probe Set ID** | **Gene Symbole** | **Cohort A FC(R/NR)** | **Cohort B FC(R/NR)** |
|---|---|---|---|---|---|---|---|
| 202422_s_at | ACSL4 | 0.30 | 0.33 | 229947_at | PI15 | 0.14 | 0.09 |
| 226517_at | BCAT1 | 0.39 | 0.47 | 220014_at | PRR16 | 0.39 | 0.42 |
| 204103_at | CCL4 | 0.38 | 0.39 | 1554997_a_at | PTGS2 | 0.21 | 0.12 |
| 219947_at | CLEC4A | 0.49 | 0.44 | 204748_at | PTGS2 | 0.22 | 0.13 |
| 231766_s_at | COL12A1 | 0.28 | 0.39 | 223809_at | RGS18 | 0.46 | 0.41 |
| 205159_at | CSF2RB | 0.42 | 0.42 | 209071_s_at | RGS5 | 0.46 | 0.49 |
| 214974_x_at | CXCL5 | 0.23 | 0.10 | 203535_at | S100A9 | 0.39 | 0.20 |
| 206336_at | CXCL6 | 0.22 | 0.25 | 220330_s_at | SAMSN1 | 0.35 | 0.43 |
| 207610_s_at | EMR2 | 0.40 | 0.37 | 1555638_a_at | SAMSN1 | 0.38 | 0.39 |
| 217967_s_at | FAM129A | 0.36 | 0.38 | 206211_at | SELE | 0.27 | 0.17 |
| 217966_s_at | FAM129A | 0.47 | 0.44 | 202627_s_at | SERPINE1 | 0.40 | 0.38 |
| 155499_s_at | FCER1G | 0.42 | 0.39 | 202498_s_at | SLC2A3 | 0.41 | 0.31 |
| 203561_at | FCGR2A | 0.39 | 0.33 | 218404_at | SNX10 | 0.49 | 0.48 |
| 1554741_s_at | FGF7 | 0.33 | 0.38 | 227697_at | SOCS3 | 0.41 | 0.29 |
| 229435_at | GLIS3 | 0.41 | 0.43 | 201858_s_at | SRGN | 0.40 | 0.36 |
| 211959_at | IGFBP5 | 0.28 | 0.43 | 204397_x_at | STC1 | 0.26 | 0.24 |
| 203424_s_at | IGFBP5 | 0.43 | 0.34 | 230746_s_at | STC1 | 0.19 | 0.21 |
| 206420_at | IGSF6 | 0.48 | 0.35 | 204393_s_at | STC1 | 0.35 | 0.28 |
| 206924_at | IL11 | 0.13 | 0.17 | 229723_at | TAGAP | 0.30 | 0.24 |
| 206172_at | IL13RA2 | 0.22 | 0.20 | 210664_s_at | TFPI | 0.50 | 0.45 |
| 205207_at | IL6 | 022 | 0.17 | 209278_s_at | TFPI2 | 0.16 | 0.15 |
| 205198_at | IL7R | 0.45 | 0.49 | 210176_at | TLR1 | 0.41 | 032 |
| 210511_s_at | INHBA | 0.23 | 0.22 | 201645_at | TNC | 0.22 | 0.32 |
| 226001_at | KLHL5 | 0.39 | 0.49 | 204933_s_at | TNFRSF11B | 0.32 | 0.23 |
| 203269_at | LCP2 | 0.40 | 0.42 | 204932_at | TNFRSF11B | 0.28 | 0.24 |
| 229937_x_at | LILRB1 | 0.42 | 0.44 | 231227_at | WNT5A | 0.25 | 0.39 |
| 210146_x_at | LILRB2 | 033 | 0.26 | 203990_s_at | WNT5A | 0.28 | 0.32 |
| 206953_s_at | LPHN2 | 0.42 | 0.45 | 213423_at | WNT5A | 0.31 | 0.32 |
| 206584_at | LY96 | 0.38 | 0.46 | 232297_at | N/A | 0.44 | 0.49 |
| 220122_at | MCTP1 | 0.44 | 0.47 | 227140_at | N/A | 0.16 | 0.17 |
| 203434_s_at | MME | 0.33 | 0.39 | 209960_at | N/A | 0.42 | 0.43 |
| 205828_at | MMP3 | 0.25 | 0.30 | 242388_x_at | N/A | 0.42 | 0.37 |
| 224940_s_at | PAPPA | 0.39 | 0.39 | 223802_at | N/A | 0.33 | 0.28 |
| 228128_x_at | PAPPA | 0.35 | 0.38 | 226218_at | N/A | 0.41 | 0.46 |
| 224941_at | PAPPA | 0.39 | 0.43 | 226847_at | N/A | 0.36 | 0.37 |
| 203708_at | PDE4B | 0.41 | 0.31 | 226237_at | N/A | 0.36 | 0.32 |
| 211302_s_at | PDE4B | 0.44 | 0.29 | 209683_at | N/A | 0.44 | 0.47 |
| FC, fold change; N/A, not available; NR, non-responders; NR, non-responders | | | | | | | |
| *Full annotation for each probe set is given in Supplementary table 1 | | | | | | | |

Ten overlapping genes came up in the top 20 significantly differentially expressed known genes for cohort A and 3 overlapping genes belonged to the top 20 genes of cohort B. The top 10 most significant biological functions that were associated with the list of the common probe sets.

To identify which biological processes and/or diseases are associated with (non-)response to infliximab treatment, a Bio Functional Analysis was performed on the significant probe sets from each LIMMA analysis (Supplementary table 3). The Bio Functional analyses showed a common predominance of the biological functions: immune response, cellular movement, cellular growth and proliferation, hematological system development and function, cell-to-cell signalling and interaction, cell death and tissue morphology/development.

### Class prediction analysis of responders and non-responders

PAM analysis was carried out on the top 20 and top 5 most significantly different known genes that were identified by LIMMA analysis between responders and non-responders in cohort A, cohort B and both cohorts combined.
In cohort A, PAM analysis of the top 20 and top 5 genes allowed classification of samples as responder and non-responder with an overall accuracy of 91.7% (22/24) and 83.3% (20/24), respectively. The top 20 and top 5 gene classifier of cohort A were used to predict the (non-)response of the samples from cohort B. Both cohort A classifiers predicted 3/12 responders (25% sensitivity) and 10/10 non-responders (100% specificity) in cohort B correctly. Hierarchical clustering of the log2 expression values from the top 20 and top 5 genes in cohort A resulted in 2 major clusters of responders versus non-responders, with two non-responders misclassified in the cluster of responders.
In cohort B, PAM analysis of the top 20 and top 5 genes that were identified by LIMMA analysis in cohort B revealed an overall accuracy of 86.4% (19/22) and 90.9% (20/22), respectively. The top 20 and top 5 gene classifiers of cohort B predicted the samples from cohort A with an overall accuracy of 66.7% and 70.8%, respectively.
For the two cohorts combined, PAM analysis of the top 20 and top 5 genes from the LIMMA analysis for both cohorts combined allowed classification of responders and non-responders with an overall accuracy of 84.8% (39/46) and 89.1% (41/46), respectively. For the two cohorts combined, PAM analysis showed that a predictive signature of 20 gene probes was not better than a panel of 5 gene probes to differentiate responders from non-responders.
The subset of the top 20 and top 5 gene classifiers used for the PAM analyses in cohort A, cohort B and both cohorts combined are listed in Supplementary Table 4. Table 4 summarizes the results (overall accuracy, sensitivity and specificity) of the PAM analyses.

**Table 4 Summary of the results from PAM analyses in cohort A, cohort B and both cohorts combined**

| **PAM analysis** | | | |
|---|---|---|---|
| **UC cohort A (R=8, NR=16)** | **Sensitivity** | **Specificity** | **Overall accuracy** |
| Top 20 genes | 100% (8/8) | 87.5% (14/16) | 91.7% |
| Top 5 genes | 75% (6/8) | 87.5% (14/16) | 83.3% (20/24) |

| **The top-ranked gene classifiers of cohort A were used to classify samples in cohort B (R=12, NA=10)** | **Sensitivity in cohort B** | **Specificity in cohort B** | **Overall accuracy in cohort B** |
|---|---|---|---|
| Cohort A top 20 genes | 25% (3/12) | 100% (10/10) | 59.1% (13/22) |
| Cohort A top 5 genes | 25% (3/12) | 100% (10/10) | 59.1% (13/22) |

| **UC cohort B (R=12, NR=10)** | **Sensitivity** | **Specificity** | **Overall accuracy** |
|---|---|---|---|
| Top 20 genes | 91.7% (11/12) | 80% (8/10) | 86.4% (19/22) |
| Top 5 genes | 91.7% (11/12) | 90% (9/10) | 90.9% (20/22) |

| **The top-ranked gene classifiers of cohort A were used to classify samples in cohort B (R=12, NR=10)** | **Sensitivity in cohort A** | **Specificity in cohort A** | **Overall accuracy in cohort A** |
|---|---|---|---|
| Cohort B top 20 genes | 87.5% (7/8) | 56.3% (9/16) | 66.7% (16/24) |
| Cohort B top 5 genes | 87.5% (7/8) | 62.5% (10/16) | 70.8% (17/24) |

| **UC cohort A and B (R=20, NR=26)** | **Sensitivity** | **Specificity** | **Overall accuracy** |
|---|---|---|---|
| Top 20 genes | 95% (19/20) | 76.9% (20/26) | 84.8% (39/46) |
| Top 5 genes | 95% (19/20) | 84.6% (22/26) | 89.1% (41/46) |
| NR, non-responders; R, responders | | | |

### Validation of the microarray data by qPCR

To confirm the microarray data, qPCR was performed for the top 5 significant known genes of the LIMMA analysis of the two cohorts combined [TNFRSF11B, STC1, PTGS2, IL-13Ralpha2 and IL-11]. LIMMA analysis in cohort A showed also significantly increased mRNA expression of these genes in untreated UC when compared to controls. Quantitative RT-PCR confirmed the differential expression of these genes between the responders, non-responders and controls in cohort A.

### DISCUSSION

There is a great need at defining molecular mechanisms that underlie (non-)response to anti-TNF-alpha therapy in UC. In this study we investigated endoscopic mucosal biopsy derived mRNA expression to identify a 'gene expression fingerprint' distinguishing primary non-responders from responders and to identify gene panels predictive of response to infliximab, a chimeric IgG1 monoclonal antibody to TNF-alpha. Two independent cohorts of patients with refractory UC who received a first treatment with infliximab were studied. The first cohort included 24 consecutive patients treated at the University Hospital in Leuven, Belgium and the second cohort consisted of 22 patients who were treated in the ACT1 clinical trial in UC. Although in the ACT1 trial clinical response was used as an endpoint for the study, we chose to use a more stringent criteria for response to infliximab in this study, i.e. complete endoscopic and histologic healing at the preset time point. We classified patients as responders if they achieved complete healing both endoscopically and histologically. All other patients were considered non-responders although some of them showed clinical improvement or showed mucosal healing at a later time point. We used mucosal healing as an endpoint as it is less open to bias than a symptom score, and there is little chance that complete mucosal healing would occur only by spontaneous disease evolution. Moreover we were able in this manner to correlate endoscopic activity with histologic activity. Combined endoscopic and histologic healing minimizes the placebo effect on the results, with the endoscopic results providing a distinct signature based upon the biological activity of the anti-TNF agent. In cohort A, endoscopic evaluation was performed in an open fashion by an endoscopist who was informed about the treatment but histology was blinded. In cohort B, both endoscopic and histologic assessment were blinded as the patients in this cohort took part in the ACT1 placebo controlled clinical trial with infliximab in UC(Rutgeerts P, Sandborn WJ, Feagan BG et al. Infliximab for induction and maintenance therapy for ulcerative colitis. N Engl J Med 2005;353(23):2462-76).
Microarray analysis of pre-treatment mucosal biopsies for both cohorts A and B yielded 179 and 323 probe sets respectively that were significantly downregulated in responders versus non-responders. Although the differentially expressed probe sets were not the same for the two cohorts, a comparative analysis of cohort A and B showed an overlap for 74 probe sets differentially expressed, representing 53 different known genes. The top biological functions that were overrepresented within the lists of significant probe sets were cellular movement, hematological system development and function, immune response and cell death. It is not surprising that there is no perfect overlap for the signatures found in both cohorts. This is likely due to differences in patient populations, different environmental background and concomitant therapies.
For each cohort, the top significant gene probes were used for predicting response to infliximab in UC and the overall accuracy with the sensitivity and specificity were calculated. The overall accuracy of the top 20 and top 5 genes for cohort A were 92% and 83%, respectively. For cohort B, overall accuracy was 86% and 91 % for the top 20 and top 5 genes, respectively. When using cohort B to validate the predictive signature obtained in cohort A, we found a 100% sensitivity but a low specificity. When using cohort A to validate the predictive signature of cohort B, we found an overall accuracy of around 70%.
We also carried out an analysis of the microarray data from cohorts A and B combined. For both cohorts combined, the number of gene probes used for prediction was reduced from an initial panel of 20 to 5 gene probes. The defined 5 gene signature panel included TNFRSF11B, STC1, PTGS2, IL-13Ralpha2 and IL-11 and predicted the response to infliximab therapy with 89% accuracy. All five proteins encoded by the five genes identified in this study are involved in signalling in the adaptive immune response, inflammation and TNF pathways(13-18). We confirmed the predictive value of each of the top 5 probes derived from the combined analysis by qPCR. Further study of the genes and pathways identified in the present studies should allow a better understanding of the molecular mechanisms of infliximab action and mechanisms of resistance to anti-TNF therapy.
We propose to further prospectively validate this gene signature in patients to whom a first anti-TNF treatment is given.
In conclusion we developed gene expression profiles in mucosal biopsies from two cohorts of infliximab naieve patients prior to infliximab therapy. Our studies demonstrate that a limited number of genes involved in the inflammatory cascade account for resistance of UC to respond to anti-TNF-alpha therapy.

### Cloning, expression and purification of a TNFα-IL13RA2 diabody

The invention provides a dual-specific ligand comprising a first immunoglobulin variable domain binding to TNFα and a second immunoglobulin variable domain binding to IL 13RA2.

Bispecific antibodies comprising complementary pairs of V _{H} and V _{L} regions are known in the art. These bispecific antibodies must comprise two pairs of V _{H} and V _{L} , each V _{H} /V _{L} pair binding to a single antigen or epitope.

Methods described involve hybrid hybridomas (Milstein & Cuello A C, Nature 305:537-40), minibodies (Hu et al., (1996) Cancer Res 56:3055-3061;), diabodies (Holliger et al., (1993) Proc. Natl. Acad. Sci. USA 90, 6444-6448; WO 94/13804), chelating recombinant antibodies (CRAbs; (Neri et al., (1995) J. Mol. Biol. 246, 367-373), biscFv (e.g. Atwell et al., (1996)Mol. Immunol. 33, 1301-1312), "knobs in holes" stabilised antibodies (Carter et al., (1997) Protein Sci. 6, 781-788).

Here, the bispecific diabody is produced by expressing two polypeptide chains with the structure V_{H}A-V_{L}B and V_{H}B-V_{L}A within the same cell in a diabody format. The small linker connecting the V_{H} and V_{L} domain to approximately 5 residues forces dimerization of the two polypeptide chains by crossover pairing of the V_{H} and V_{L} domains.

### Purification of RNA and amplification of variable domains

Total RNA from 10⁶ cells of the mouse hybridoma anti-TNFα (A) and IL13RA2 (B) was extracted using the RNeasy mini kit (Qiagen). mRNA was isolated from the total RNA preparation with the Oligotex mRNA kit (Qiagen). The complementary DNA (cDNA) was synthesized using an RT-PCR Kit (Roche Diagnostics) using oligo dT as primer. The polymerase chain reaction technique (PCR) for the specific amplification of the heavy and light chain variable domain genes was used. The employed synthetic primers were designed on the basis of the consensus sequences for mouse IgG and kappa chains, reported by Kabat E. et al. (US Department of Health and Human Services, NIH, 1991).

For PCR, the following conditions are used: denaturizing to 94° C., 1 minute, annealing to 55° C., 1 minute, extension to 72° C., 1 minute, 25 cycles, with 5 additional minutes of extension to the temperature already described in the last cycle, everything in an Eppendorf Mastercycle machine. The final volumes of each reaction were 100 µL. All the oligonucleotides were used to a final concentration of 1 µM.

The DNA amplified fragments were purified from agarose gels using the QIAquick Gel Extraction Kit (Qiagen), and were cloned independently in the pPCR-script vector (Stratagene).

### Nucleotide Sequence of the Variable Domains

The nucleotide sequence of the light and heavy chain variables domains cloned in the pPCR-script vector is determined by means of automated methods using commercially available kits.

pPCR-script derived plasmids, pV_{H}A, pVLA and pV_{H}B, pV_{H}B, are selected as containing the correct sequence for the V_{H} and V_{L} of anti-TNF (A) and V_{H} and V_{L} of anti-IL13RA2 (B), respectively.

### Assembly of the diabody

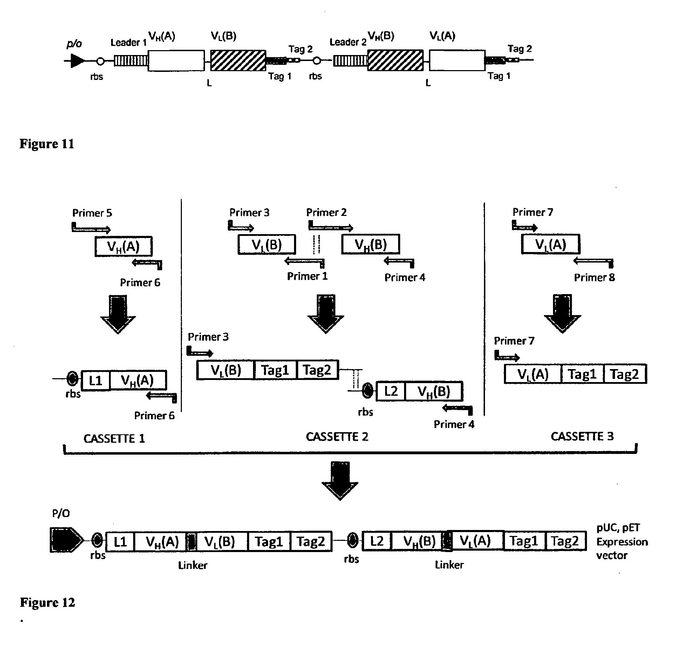

Different expression plasmids are available and derived from commercially available vectors such as pUC, pET28, pET20b,... Plasmids should only allow the construction of the following cassette (Figure 11). To obtain this final cassette allowing expression of the diabody in a bacterial host cell, a multistep cloning procedure is necessary. Many different approaches are possible. An example is given below (Figure 12).

Assembly of the V_{L}(B) and V_{H}(B) domains was performed by SOE-PCR (splicing by overlap extension) (McGuinness *et al.,* 1996; Clackson *et al.,* 1991). In short: Final V_{L}(B) and V_{H}(B) PCR products are assembled by 9 PCR cycles without, followed by 25 cycles with pull-through primers 3 (containing linker (AKTTPKLGG) encoding sequence) and 4 (containing linker encoding sequence) (Figure 12). Primers 1 and 2 are partially complementary and comprise sequences encoding Tag 1, Tag 2 and an appropriate restriction site (primer 1) and sequence encoding an rbs, leader sequence and an appropriate restriction site (primer 2). Double digestion of the resulting resulting assembled PCR product yields cassette 2.

V_{H}(A) and V_{L}(A) domains are PCR amplified from pHA and pVA, respectively, using primer 5 (containing rbs, leader sequence and appropriate restriction site) and primer 6 (containing linker encoding sequence and identical restriction site as in primer primer 3) and primer 7 (containing linker encoding sequence and identical restriction site as primer 4) and primer 8 (containing sequences encoding Tag 1 and Tag2 and an appropriate restriction site), respectively. Double digestion of the obtained PCR products yields cassette 1 [V_{H}(A)] and cassette 3 [V_{L}(A)], respectively.

Cassettes 1,2 and 3 are ligated together in the respective expression vector downstream of the promoter and operator site to driving secretion and subsequent assembly of the diabody in the periplasmic space.

### Diabody expression and purification

For functional expression of the bispecific diabody in the bacterial periplasm, the obtained expression vector encoding the TNFα-IL13RA2 diabody is transformed into E. *coli* K12 strain RV308 (Δ*lac*.^{χ}74*gal*ISII::OP308*strA*). Transformed bacteria are grown overnight in shake flasks containing 2YT medium with 0.1 g/L ampicillin and 100 mM glucose (2YT_{GA}) at 26°C. Dilutions (1/50) of the overnight cultures in 2YT_{GA} are grown as flask cultures at 26°C with shaking at 200 rpm. At OD₆₀₀ = 0.7, bacteria are harvested by centrifugation and resuspended in the same volume of YTBS medium (2YT containing 1 M sorbitol and 2.5 mM glycine betaine). Isopropyl ß-D-thiogalactoside is added to a final concentration of 0.2 mM, and growth was continued at 23°C for 13 h. The bacterial cells are then harvested by centrifugation, and periplasmic extracts were isolated as previously described.

The periplasmic extract is passed through a filter of pore size 0.2µm and dialyzed against 20 mmol/L Tris-HCl, 0.5 mol/L NaCl, pH 7.9 and purified as described before.

Due to the presence of a 6His Tag, purification is achieved by immobilized metal affinity chromatography (IMAC) on Ni²⁺-charged chelating agarose (GE Healthcare) following the manufacturer's recommendations

The term "pharmaceutically acceptable" is used herein to mean that the modified noun is appropriate for use in a pharmaceutical product.

As used herein, the term "pharmaceutically acceptable carrier" includes also any and all solvents, dispersion media, coatings, antibacterial and antifungal agents, isotonic agents, absorption delaying agents, and the like. The use of such media and agents for pharmaceutically active substances is well known in the art. Except insofar as any conventional media or agent is incompatible with the compositions of this invention, its use in the therapeutic formulation is contemplated. Supplementary active ingredients can also be incorporated into the pharmaceutical formulations.

The term "treatment" refers to any process, action, application, therapy, or the like, wherein a mammal, including a human being, is subject to medical aid with the object of improving the mammal's condition, directly or indirectly. In the current invention "treatment" also refers to prevention of an IBD for instance an UC or a CD.

Suppression means that IL-13Ralpha2 activation, IL-13Ralpha2 activity or IL-13Ralpha2 expression occurs for at least 20%, 30%, 40%, 50%, 60%, 70%, 80%, 90% or even 100% less than compared with the mammal as compared with the mammal not treated with an inhibitor of IL-13Ralpha2 of the invention.

The invention provides the use of a compound that inhibits the expression and/or activity of an IL-13Ralpha2 for the manufacture of a medicament for increasing the efficiency an anti-TNFalpha treatment or IBD, in particular UC or CD.

The term 'a compound that inhibits the expression' refers here to gene expression and thus to the inhibition of gene transcription and/or translation of a gene transcript (mRNA) such as for example the IL-13Ralpha2 or IL-13Ralpha2 mRNA. Preferably said inhibition is at least 20%, 30%, 40%, 50%, 60%, 70%, 80%, 90% or even higher. The term 'a compound that inhibits the activity' refers here to the protein that is produced such as the IL-13Ralpha2 protein. Said inhibition of activity leads to a diminished interaction of IL-13Ralpha2 with its substrates and a diminished IL-13Ralpha2 activity where under the catalyzed DNA degradation and an inhibition of the IL-13Ralpha2 dependent inhibition of responding on an anti-TNFalpha IBD treatment. Preferably said inhibition of IL-13Ralpha2 is at least 20%, 30%, 40%, 50%, 60%, 70%, 80%, 90% or even higher.

The present disclosure shows that response to anti-TNFalpha is in an IBD treatment significantly increased if of IL-13Ralpha2 is inhibited and that response to an anti-TNF treatment can be increased by the usage of inhibitors of IL-13Ralpha2.

Thus more specifically the invention also relates to molecules that neutralize the activity of IL-13Ralpha2 by interfering with its synthesis, translation, dimerisation, substrate-binding. By molecules it is meant peptides, peptide aptamers, tetrameric peptides, proteins, organic molecules, soluble substrates of IL-13Ralpha2 and any fragment or homologue thereof having the same neutralizing effect as stated above. Also, the invention the molecules comprise antagonists of IL-13Ralpha2 such as anti- of IL-13Ralpha2 antibodies and functional fragments derived thereof, anti-sense RNA and DNA molecules and ribozymes that function to inhibit the translation of IL-13Ralpha2, all capable of interfering/or inhibiting of the IL-13Ralpha2 -dependent pathways.

By synthesis it is meant transcription of IL-13Ralpha2. Small molecules can bind on the promoter region of IL-13Ralpha2 and inhibit binding of a transcription factor or said molecules can bind said transcription factor and inhibit binding to the IL-13Ralpha2 - promoter.

By of IL-13Ralpha2 it is meant also its isoforms, which occur as a result of alternative splicing, and allelic variants thereof.

Antagonists of of IL-13Ralpha2 can increase the anti-TNFapha treatment response in said IBD and in particular in UC or CD...

The term 'antibody' or 'antibodies' relates to an antibody characterized as being specifically directed against of IL-13Ralpha2 or any functional derivative thereof, with said antibodies being preferably monoclonal antibodies; or an antigen-binding fragment thereof, of the F(ab')2, F(ab) or single chain Fv type, of the single domain antibody type or any type of recombinant antibody derived thereof. These antibodies of the invention, including specific polyclonal antisera prepared against of IL-13Ralpha2, or any functional derivative thereof, have no cross-reactivity to others proteins. The monoclonal antibodies of the invention can for instance be produced by any hybridoma liable to be formed according to classical methods from splenic cells of an animal, particularly of a mouse or rat immunized against of IL-13Ralpha2 or any functional derivative thereof, and of cells of a myeloma cell line, and to be selected by the ability of the hybridoma to produce the monoclonal antibodies recognizing of IL-13Ralpha2 or any functional derivative thereof which have been initially used for the immunization of the animals. The monoclonal antibodies according to this embodiment of the invention may be humanized versions of the mouse monoclonal antibodies made by means of recombinant DNA technology, departing from the mouse and/or human genomic DNA sequences coding for H and L chains or from cDNA clones coding for H and L chains. Alternatively the monoclonal antibodies according to this embodiment of the invention may be human monoclonal antibodies. Such human monoclonal antibodies are prepared, for instance, by means of human peripheral blood lymphocytes (PBL) repopulation of severe combined immune deficiency (SCID) mice as described in PCT/EP 99/03605 or by using transgenic non-human animals capable of producing human antibodies as described in US patent 5,545,806. Also fragments derived from these monoclonal antibodies such as Fab, F(ab)'2 and ssFv ("single chain variable fragment"), providing they have retained the original binding properties, form part of the present invention. Such fragments are commonly generated by, for instance, enzymatic digestion of the antibodies with papain, pepsin, or other proteases. It is well known to the person skilled in the art that monoclonal antibodies, or fragments thereof, can be modified for various uses. The antibodies involved in the invention can be labeled by an appropriate label of the enzymatic, fluorescent, or radioactive type.

Small molecules, e.g. small organic molecules, and other drug candidates can be obtained, for example, from combinatorial and natural product libraries.

Random peptide libraries, such as the use of tetrameric peptide libraries such as described in WO0185796, consisting of all possible combinations of amino acids attached to a solid phase support, or such as a combinatorial library of peptide aptamers, which are proteins that contain a conformationally constrained peptide region of variable sequence displayed from scaffold as described in Colas et al. Nature 380: 548-550, 1996 and Geyer et al., Proc. Natl. Acad. Sco. USA 96: 8567-8572, 1999, may be used in the present invention.

Also transdominant-negative mutant forms of IL-13Ralpha2 -ligands can be used to inhibit of IL-13Ralpha2 dependent pathways.

Also within the scope of the invention is the use of oligoribonucleotide sequences, that include anti-sense RNA and DNA molecules and ribozymes that function to inhibit the translation of IL-13Ralpha2 mRNA. Anti-sense RNA and DNA molecules act to directly block the translation of mRNA by binding to targeted mRNA and preventing protein translation. In regard to antisense DNA, oligodeoxyribonucleotides derived from the translation initiation site.

Ribozymes are enzymatic RNA molecules capable of catalyzing the specific cleavage of RNA. The mechanism of ribozyme action involves sequence specific hybridization of the ribozyme molecule to complementary target RNA, followed by a endonucleolytic cleavage. Within the scope of the invention are engineered hammerhead motif ribozyme molecules that specifically and efficiently catalyze endonucleolytic cleavage of IL-13Ralpha2 sequences. Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features such as secondary structure that may render the oligonucleotide sequence unsuitable.

Both anti-sense RNA and DNA molecules and ribozymes of the invention may be prepared by any method known in the art for the synthesis of RNA molecules. These include techniques for chemically synthesizing oligodeoxyribonucleotides well known in the art such as for example solid phase phosphoramidite chemical synthesis. Alternatively, RNA molecules may be generated by in vitro and in vivo transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize anti-sense RNA constitutively or inducibly, depending on the promoter used, can be introduced stably into cell lines.

The present invention provides an in vitro method of diagnosing for predicting if a subject suffering of an inflammatory bowel disease will respond to an anti-TNFα therapy, such method comprisese: (**a**) analyzing the level of IL-13Ralpha2 expression or activity of expression product in a biological sample isolated from said subject, and (**b**) compare said level of expression or activity with the 13Ralpha2 expression or activity in a control sample; whereby a different level of IL-13Ralpha2 expression or activity relative to a control sample is an indication of response to anti-TNFα therapy or a propensity thereto. A decreased level of IL-13Ralpha2 is indicative of a positive response thereto. The in vitro method of diagnosing for predicting if a subject suffering of an inflammatory bowel disease will respond to an anti-TNFα therapy is particular suitable for patients affected by IBD, such CD or UC.

In particular the present invention provides an in vitro method of diagnosing for predicting if a subject suffering of an inflammatory bowel disease will respond to an anti-TNFα antibody therapy such as a therapy with an anti TNFα therapeutic antibody for instance an antibody that blocks the action of TNFα by preventing it from binding to its receptor in the cell such as Infliximab, Adalimumab or Etanercept, such method comprising: (**a**) analyzing the level of IL-13Ralpha2 expression or activity of expression product in a biological sample isolated from said subject, and (**b**) compare said level of expression or activity with the 13Ralpha2 expression or activity in a control sample; whereby a different level of IL-13Ralpha2 expression or activity relative to a control sample is an indication of response to anti-TNFα therapy or a propensity thereto. A decreased level of IL-13Ralpha2 is indicative of a positive response thereto and is predictive for the responders.

In yet a more specific embodiment of the present invention provides an in vitro method of diagnosing for predicting if a subject suffering of an ulcerative colitis will respond to an anti-TNFα antibody therapy such as a therapy with an anti TNFα therapeutic antibody for instance an antibody that blocks the action of TNFα by preventing it from binding to its receptor in the cell such as Infliximab, Adalimumab or etanercept, such method comprising: (**a**) analyzing the level of IL-13Ralpha2 expression or activity of expression product in a biological sample isolated from said subject, and (**b**) compare said level of expression or activity with the 13Ralpha2 expression or activity in a control sample; whereby a different level of IL-13Ralpha2 expression or activity relative to a control sample is an indication of response to anti-TNFα therapy or a propensity thereto. A decreased level of IL-13Ralpha2 is indicative of a positive response thereto and is indicative for the responders.

The present invention furthermore concerns an in vitro method of diagnosis to predict the responding or non responding of a subject on an anti-TNFalpha treatment of IBD, or a propensity thereto in a subject, said method (**a**) obtaining an expression profile in a biological sample isolated from said subject, wherein said expression profile consists of the analysis of the level of IL-13Ralpha2 expression or activity of an IL-13Ralpha2 expression product in combination with the gene expression level or activity of a gene product of at least one gene selected from the group consisting of TNFRSF11B, STC1, PTGS2 and IL-11; and (**b**) comparing said obtained expression profile to a reference expression profile to determine whether said sample is from subject having a inflammatory bowel disease phenotype or a propensity thereto. In this in vitro method the expression profile can consists of any one of the following combinations; IL-13Ralpha2 and TNFRSF11B; IL-13Ralpha2 and STC1; IL-13Ralpha2 and PTGS2; IL-13Ralpha2 and IL-11; IL-13Ralpha2 and STC1 and PTGS2; IL-13Ralpha2 and TNFRSF11B and PTGS2; IL-13Ralpha2 and TNFRSF11B and STC1; IL-13Ralpha2 and IL-11 and TNFRSF11B; IL-13Ralpha2 and IL-11 and STC1; IL-13Ralpha2 and IL-11 and PTGS2; IL-13Ralpha2 and TNFRSF11B and PTGS2 and STC1; IL-13Ralpha2 and IL-11 and PTGS2 and STC1; IL-13Ralpha2 and TNFRSF11B and IL-11 and STC1; IL-13Ralpha2 and TNFRSF11B and PTGS2 and IL-11.

The present invention furthermore concerns an in vitro method of diagnosis to predict the responding or non responding of a subject on an anti-TNFalpha treatment of **IBD**, or a propensity thereto in a subject, said method (**a**) obtaining an expression profile in a biological sample isolated from said subject, wherein said expression profile consists of the analysis of the level of IL-13Ralpha2 expression or activity of an IL-13Ralpha2 expression product in combination with the gene expression level or activity of a gene product of at least two gene selected from the group consisting of TNFRSF11B, STC1, PTGS2 and IL-11; and (**b**) comparing said obtained expression profile to a reference expression profile to determine whether said sample is from subject having a inflammatory bowel disease phenotype or a propensity thereto.

The present invention furthermore concerns an in vitro method of diagnosis to predict the responding or non responding of a subject on an anti-TNFalpha treatment of **IBD**, or a propensity thereto in a subject, said method (**a**) obtaining an expression profile in a biological sample isolated from said subject, wherein said expression profile consists of the analysis of the level of IL-13Ralpha2 expression or activity of an IL-13Ralpha2 expression product in combination with the gene expression level or activity of a gene product of at least three gene selected from the group consisting of TNFRSF11B, STC1, PTGS2 and IL-11; and (**b**) comparing said obtained expression profile to a reference expression profile to determine whether said sample is from subject having a inflammatory bowel disease phenotype or a propensity thereto.

The present invention furthermore concerns an in vitro method of diagnosis to predict the responding or non responding of a subject on an anti-TNFalpha treatment of **IBD**, or a propensity thereto in a subject, said method (**a**) obtaining an expression profile in a biological sample isolated from said subject, wherein said expression profile consists of the analysis of the level of IL-13Ralpha2 expression or activity of an expression product of at the gene cluster of the genes IL-13Ralpha2, TNFRSF11B, STC1, PTGS2 and IL-11; and (**b**) comparing said obtained expression profile to a reference expression profile to determine whether said sample is from subject having a inflammatory bowel disease phenotype or a propensity thereto.

The expression product of any of the previously described in vitro method can be a nucleic acid molecule selected from the group consisting of mRNA and cDNA mRNA or derived polypeptides. The sample in any of the previously described in vitro method can be isolated form said subject is selected from a group consisting of a) a liquid containing cells; (b) a tissue-sample; (c) a cell-sample and (a) a cell biopsy for instance obtainable by a colonic mucosal biopsy.

The in-vitro method according to any one of the previous methods here above described can in a particular embodiment comprise the detection of the level of the nucleic acids or polypeptides carried out using at least one binding agent specifically binding to the nucleic acids or polypeptides to be detected. The binding agent can be detectably labeled. The label can be selected from the group consisting of a radioisotope, a bioluminescent compound, a chemiluminescent compound, a fluorescent compound, a metal chelate, biotin, digoxygenin and an enzyme.

In a particular specific situation of this embodiment at least one binding agent being a nucleic acid hybridising to a nucleic acid is used for the detection of the marker molecules, in particular for the detection of IL-13Ralpha2, TNFRSF11B, STC1, PTGS2 and IL-11 expression. Such method can further comprise the detection reaction comprising a nucleic acid amplification reaction. The method can be used for in-situ detection

The present invention provides an in vitro method of diagnosing for predicting if a subject suffering of an inflammatory bowel disease will respond to an anti-TNFα therapy, such method comprises: (a) analyzing the level of IL-13Ralpha2 expression or activity of expression product in a biological sample isolated from said subject, and (b) compare said level of expression or activity with the 13Ralpha2 expression or activity in a control sample; whereby a different level of IL-13Ralpha2 expression or activity relative to a control sample is an indication of response to anti-TNFα therapy or a propensity thereto. A decreased level of IL-13Ralpha2 is indicative of a positive response thereto. The in vitro method of diagnosing for predicting if a subject suffering of an inflammatory bowel disease will respond to an anti-TNFα therapy is particular suitable for patients affected by inflammatory bowel disease (IBD) such as Crohn's disease (CD) or ulcerative colitis (UC).

In particular the present invention provides an in vitro method of diagnosing for predicting if a subject suffering of inflammatory bowel disease (IBD) such as Crohn's disease (CD) or ulcerative colitis (UC) will respond to an anti-TNFα antibody therapy such as a therapy with an anti TNFα therapeutic antibody for instance an antibody that blocks the action of TNFα by preventing it from binding to its receptor in the cell such as Infliximab, Adalimumab or Etanercept, such method comprising: (a) analyzing the level of IL-13Ralpha2 expression or activity of expression product in a biological sample isolated from said subject, and (b) compare said level of expression or activity with the 13Ralpha2 expression or activity in a control sample; whereby a different level of IL-13Ralpha2 expression or activity relative to a control sample is an indication of response to anti-TNFα therapy or a propensity thereto. A decreased level of IL-13Ralpha2 is indicative of a positive response thereto and is predictive for the responders.
In yet a more specific embodiment of the present invention provides an in vitro method of diagnosing for predicting if a subject suffering of inflammatory bowel disease (IBD) such as Crohn's disease (CD) or ulcerative colitis (UC) will respond to an anti-TNFα antibody therapy such as a therapy with an anti TNFα therapeutic antibody for instance an antibody that blocks the action of TNFα by preventing it from binding to its receptor in the cell such as Infliximab, Adalimumab or etanercept, such method comprising: (a) analyzing the level of IL-13Ralpha2 expression or activity of expression product in a biological sample isolated from said subject, and (b) compare said level of expression or activity with the 13Ralpha2 expression or activity in a control sample; whereby a different level of IL-13Ralpha2 expression or activity relative to a control sample is an indication of response to anti-TNFα therapy or a propensity thereto. A decreased level of IL-13Ralpha2 is indicative of a positive response thereto and is indicative for the responders.

The present invention furthermore concerns an in vitro method of diagnosis to predict the responding or non responding of a subject on an anti-TNFalpha treatment of inflammatory bowel disease (IBD) such as Crohn's disease (CD) or ulcerative colitis (UC), or a propensity thereto in a subject, said method (a) obtaining an expression profile in a biological sample isolated from said subject, wherein said expression profile consists of the analysis of the level of IL-13Ralpha2 expression or activity of an IL-13Ralpha2 expression product in combination with the gene expression level or activity of a gene product of at least one gene selected from the group consisting of TNFRSF11B, STC1, PTGS2 and IL-11; and (b) comparing said obtained expression profile to a reference expression profile to determine whether said sample is from subject having a inflammatory bowel disease (IBD) such as Crohn's disease (CD) or ulcerative colitis (UC) phenotype or a propensity thereto. In this in vitro method the expression profile can consists of any one of the following combinations; IL-13Ralpha2 and TNFRSF11B; IL-13Ralpha2 and STC1; IL-13Ralpha2 and PTGS2; IL-13Ralpha2 and IL-11; IL-13Ralpha2 and STC1 and PTGS2; IL-13Ralpha2 and TNFRSF11B and PTGS2; IL-13Ralpha2 and TNFRSF11B and STC1; IL-13Ralpha2 and IL-11 and TNFRSF11B; IL-13Ralpha2 and IL-11 and STC1; IL-13Ralpha2 and IL-11 and PTGS2; IL-13Ralpha2 and TNFRSF11B and PTGS2 and STC1; IL-13Ralpha2 and IL-11 and PTGS2 and STC1; IL-13Ralpha2 and TNFRSF11B and IL-11 and STC1; IL-13Ralpha2 and TNFRSF11B and PTGS2 and IL-11.
The present invention furthermore concerns an in vitro method of diagnosis to predict the responding or non responding of a subject on an anti-TNFalpha treatment of inflammatory bowel disease (IBD) such as Crohn's disease (CD) or ulcerative colitis (UC), or a propensity thereto in a subject, said method (a) obtaining an expression profile in a biological sample isolated from said subject, wherein said expression profile consists of the analysis of the level of IL-13Ralpha2 expression or activity of an IL-13Ralpha2 expression product in combination with the gene expression level or activity of a gene product of at least two gene selected from the group consisting of TNFRSF11B, STC1, PTGS2 and IL-11; and (b) comparing said obtained expression profile to a reference expression profile to determine whether said sample is from subject having a inflammatory bowel disease phenotype or a propensity thereto.
The present invention furthermore concerns an in vitro method of diagnosis to predict the responding or non responding of a subject on an anti-TNFalpha treatment of inflammatory bowel disease (IBD) such as Crohn's disease (CD) or ulcerative colitis (UC), or a propensity thereto in a subject, said method (a) obtaining an expression profile in a biological sample isolated from said subject, wherein said expression profile consists of the analysis of the level of IL-13Ralpha2 expression or activity of an IL-13Ralpha2 expression product in combination with the gene expression level or activity of a gene product of at least three gene selected from the group consisting of TNFRSF11B, STC1, PTGS2 and IL-11; and (b) comparing said obtained expression profile to a reference expression profile to determine whether said sample is from subject having a inflammatory bowel disease (IBD) such as Crohn's disease (CD) or ulcerative colitis (UC)phenotype or a propensity thereto.
The present invention furthermore concerns an in vitro method of diagnosis to predict the responding or non responding of a subject on an anti-TNFalpha treatment of inflammatory bowel disease (IBD) such as Crohn's disease (CD) or ulcerative colitis (UC), or a propensity thereto in a subject, said method (a) obtaining an expression profile in a biological sample isolated from said subject, wherein said expression profile consists of the analysis of the level of IL-13Ralpha2 expression or activity of an expression product of at the gene cluster of the genes IL-13Ralpha2, TNFRSF11B, STC1, PTGS2 and IL-11; and (b) comparing said obtained expression profile to a reference expression profile to determine whether said sample is from subject having a inflammatory bowel disease (IBD) such as Crohn's disease (CD) or ulcerative colitis (UC)phenotype or a propensity thereto.
The expression product of any of the previously described in vitro method can be a nucleic acid molecule selected from the group consisting of mRNA and cDNA mRNA or derived polypeptides. The sample in any of the previously described in vitro method can be isolated form said subject is selected from a group consisting of a) a liquid containing cells; (b) a tissue-sample; (c) a cell-sample and (a) a cell biopsy for instance obtainable by a colonic mucosal biopsy.
The in-vitro method according to any one of the previous methods here above described can in a particular embodiment comprise the detection of the level of the nucleic acids or polypeptides carried out using at least one binding agent specifically binding to the nucleic acids or polypeptides to be detected. The binding agent can be detectably labelled. The label can be selected from the group consisting of a radioisotope, a bioluminescent compound, a chemiluminescent compound, a fluorescent compound, a metal chelate, biotin, digoxygenin and an enzyme.
In a particular specific situation of this embodiment at least one binding agent being a nucleic acid hybridising to a nucleic acid is used for the detection of the marker molecules, in particular for the detection of IL-13Ralpha2, TNFRSF11B, STC1, PTGS2 and IL-11 expression. Such method can further comprise the detection reaction comprising a nucleic acid amplification reaction. The method can be used for in-situ detection.

Another embodiment of present invention is for use in diagnosing a subject for responsiveness on an anti-TNFalpha treatment of inflammatory bowel disease (IBD) such as Crohn's disease (CD) or ulcerative colitis (UC), or for use in monitoring the effectiveness of therapy of ulcerative colitis in patients receiving an anti TNFalpha therapy comprising:
- a) nucleic acids encoding the 13Ralpha2, TNFRSF11B, STC1, PTGS2 and IL-11 protein;
- b) reagents useful for monitoring the expression level of the one or more nucleic acids or proteins encoded by the nucleic acids of step a);
- c) instructions for use of the kit.

Another embodiment of present invention is for use in diagnosing a subject for responsiveness on an anti-TNFalpha treatment of ulcerative colitis, or for use in monitoring the effectiveness of therapy of ulcerative colitis in patients receiving an anti TNFalpha therapy comprising:
- a) one or more nucleic acids encoding one or more of the proteins selected from the group consisting of 13Ralpha2, TNFRSF11B, STC1, PTGS2 and IL-11;
- b) reagents useful for monitoring the expression level of the one or more nucleic acids or proteins encoded by the nucleic acids of step a);
- c) instructions for use of the kit.

Another embodiment of present invention is for use in diagnosing' a subject for responsiveness on an anti-TNFalpha treatment of ulcerative colitis, or for use in monitoring the effectiveness of therapy of ulcerative colitis in patients receiving an anti TNFalpha therapy comprising:
- a) one or more nucleic acids encoding two or more of the proteins selected from the group consisting of 13Ralpha2, TNFRSF11B, STC1, PTGS2 and IL-11;
- b) reagents useful for monitoring the expression level of the one or more nucleic acids or proteins encoded by the nucleic acids of step a);
- c) instructions for use of the kit.

Another embodiment of present invention is for use in diagnosing a subject for responsiveness on a anti-TNFalpha treatment of inflammatory bowel disease (IBD) such as Crohn's disease (CD) or ulcerative colitis (UC), or for use in monitoring the effectiveness of therapy of inflammatory bowel disease (IBD) such as Crohn's disease (CD) or ulcerative colitis (UC)in patients receiving an anti TNFalpha therapy comprising:
- a) one or more nucleic acids encoding three or more of the proteins selected from the group consisting of 13Ralpha2, TNFRSF11B, STC1, PTGS2 and IL-11;
- b) reagents useful for monitoring the expression level of the one or more nucleic acids or proteins encoded by the nucleic acids of step a);
- c) instructions for use of the kit.

Another embodiment of present invention is for use in diagnosing a subject for responsiveness on a anti-TNFalpha treatment of inflammatory bowel disease (IBD) such as Crohn's disease (CD) or ulcerative colitis (UC), or for use in monitoring the effectiveness of therapy of inflammatory bowel disease (IBD) such as Crohn's disease (CD) or ulcerative colitis (UC)in patients receiving an anti TNFalpha therapy comprising:
- a) one or more nucleic acids encoding four or more of the proteins selected from the group consisting of 13alpha2, TNFRSF11B, STC11, PTGS2 and IL-11;
- b) reagents useful for monitoring the expression level of the one or more nucleic acids or proteins encoded by the nucleic acids of step a);
- c) instructions for use of the kit.

Another embodiment of present invention is for use in diagnosing a subject for responsiveness on a anti-TNFalpha treatment of inflammatory bowel disease (IBD) such as Crohn's disease (CD) or ulcerative colitis (UC), or for use in monitoring the effectiveness of therapy of inflammatory bowel disease (IBD) such as Crohn's disease (CD) or ulcerative colitis (UC) in patients receiving an anti TNFalpha therapy comprising:
- a) nucleic acids encoding the proteins selected from the group consisting of 13Ralpha2, TNFRSF11B, STC11, PTGS2 and IL-11;
- b) reagents useful for monitoring the expression level of the one or more nucleic acids or proteins encoded by the nucleic acids of step a);
- c) instructions for use of the kit.

### Legend to the figures

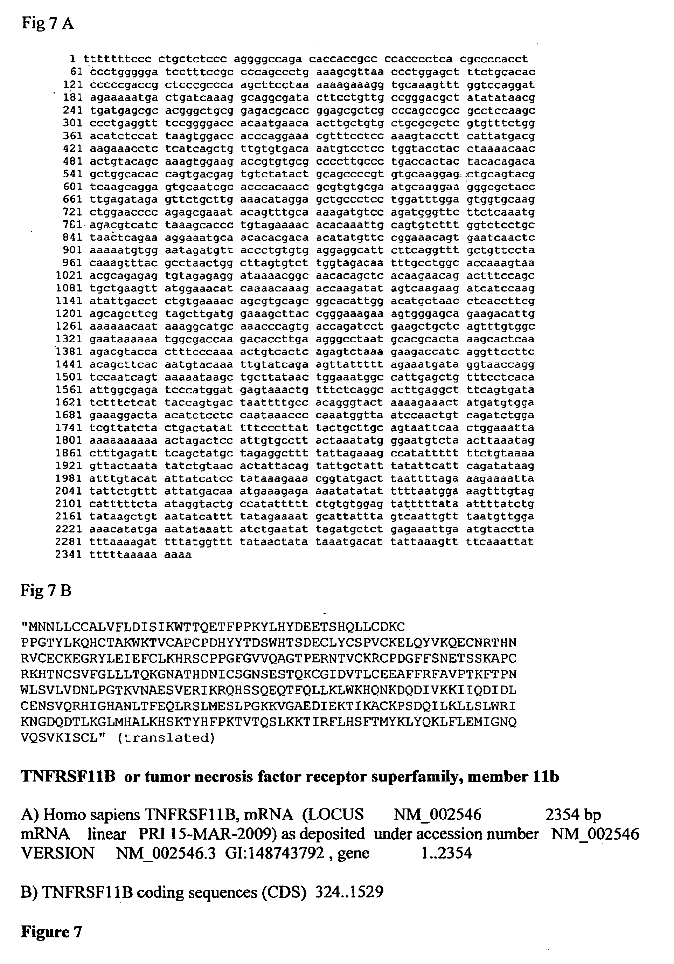

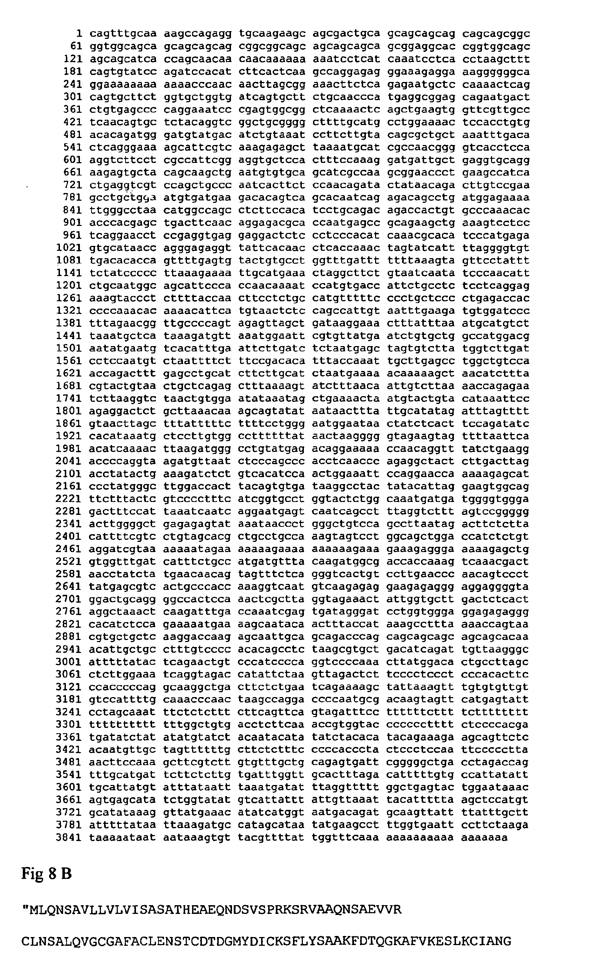

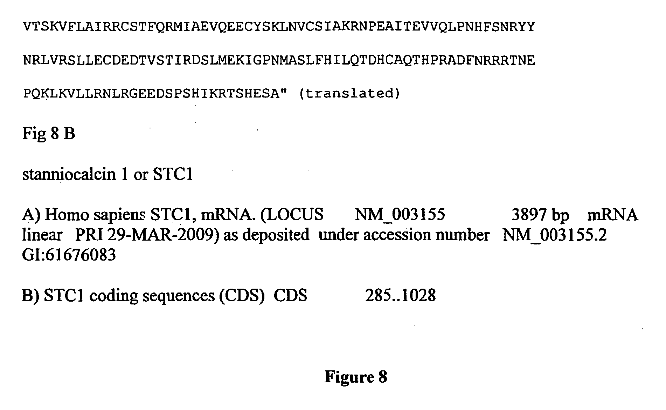

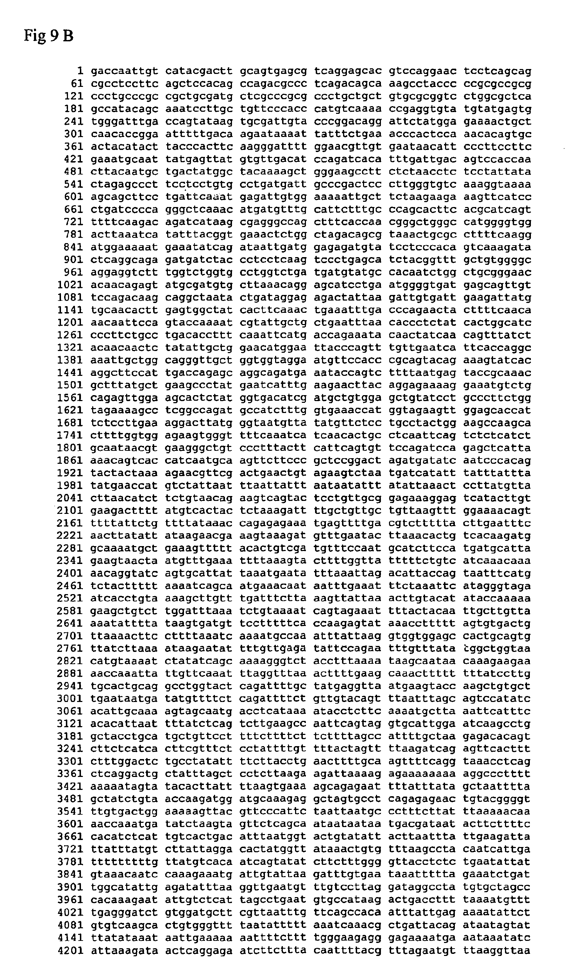

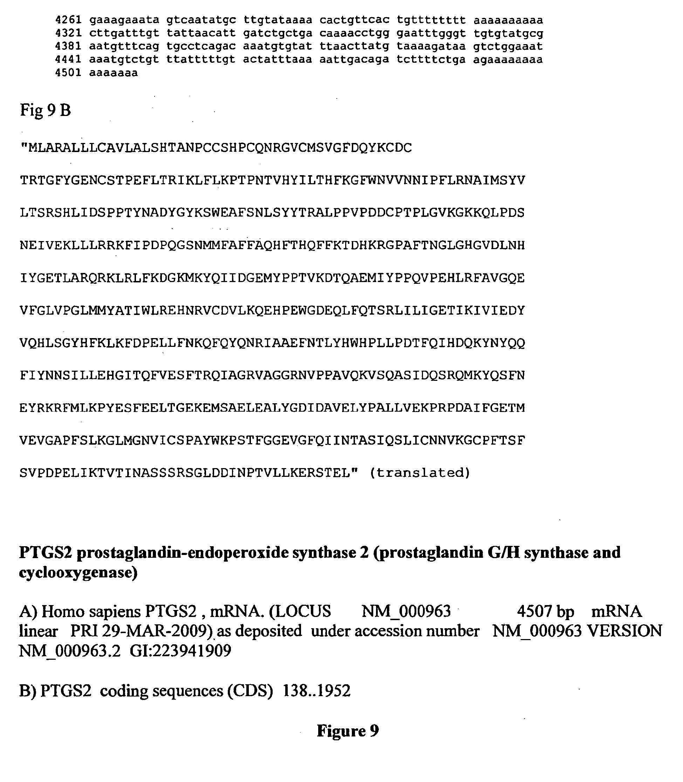

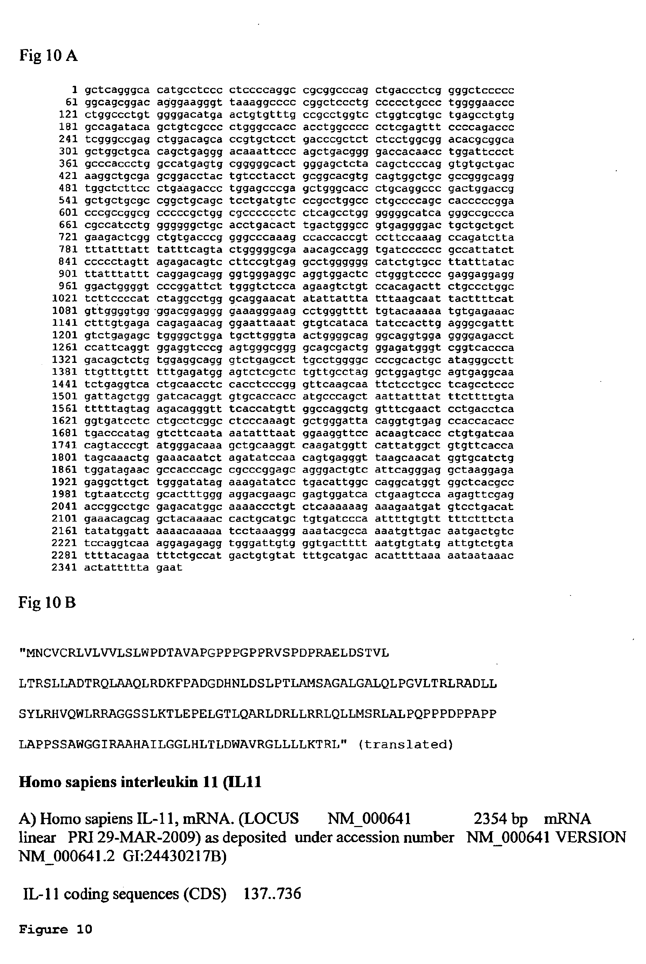

Figure 1 provides an hierarchical cluster analysis of R and NR using all DE probe sets in UC (a), CD (b) and IBD (d); as well as the top 20 significantly DE probe sets in CD (c) and IBD (e). Individual samples are shown in columns and genes in rows. The log2 expression values for individual probe sets are indicated by colour (in this print grey scales), as shown in the scale, white yellow (light) indicating a high level of expression and blue (darker) a low level of expression.
Figure 2 is a graphics of the probe set that discriminate R from NR in colonic IBD with an overall ME of 0.046 (2/43), identification by PAM. The γ-axis shows the log2 expression values of each patient for the probe sets. Red and blue circled symbols represent NR and R samples, respectively. The dot line represents the separation between R and NR.
Figure3 displays expression of IL-13Ralpha2 in IBD patients before treatment and controls, identified by micro array analysis (a) and quantitative RT-PCR (b). The horizontal lines indicate the average of each group.
Figure 4 displays IL-13Ralpha2 immunohistochemistry (a) an ovarian serous adenocarcinoma, (b) normal colon; (c) ulcerative colitis before IFX treatment; (d) Crohn's colitis before IFX treatment (original magnification, OMs x 400 for a; x 200 for b x 50 for c and d).
Figure 5 demonstrates a positive linear correlation between the TGF-betal probe set 203085_S_at and the IL_13Ralpha2 probe set, based on the log2 expression values from R and NR in IBD before treatment and controls.

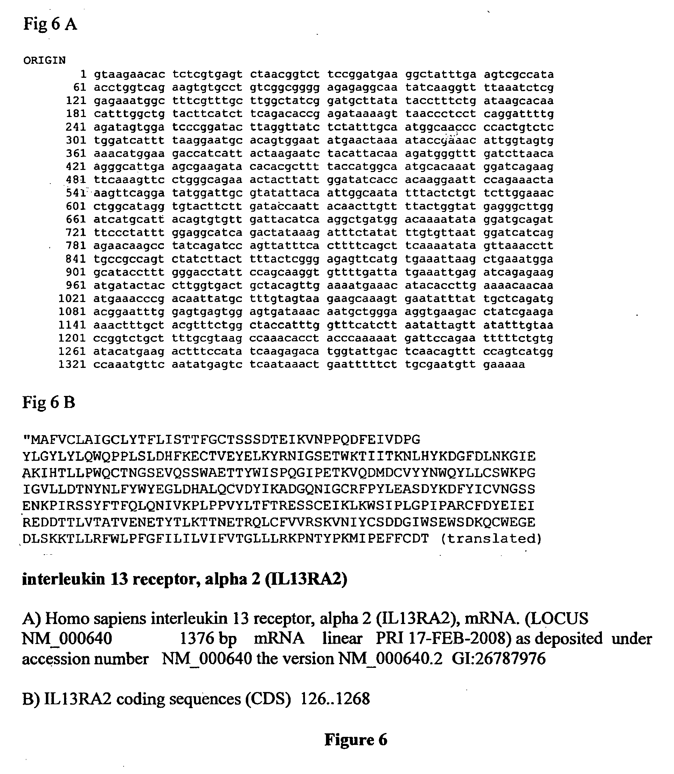

Figure 6 provides the nucleotide and protein sequence of interleukin 13 receptor, alpha 2 (IL 13RA2)
   A) Homo sapiens interleukin 13 receptor, alpha 2 (IL13RA2), mRNA. (LOCUS NM_000640 1376 bp mRNA linear PRI 17-FEB-2008) as deposited under accession number NM_000640 the version NM_000640.2 GI:26787976
   B) IL13RA2 coding sequences (CDS) 126..1268
Figure 7 provides the nucleotide and protein sequence of TNFRSF11B or tumor necrosis factor receptor superfamily, member 11b
   A) Homo sapiens TNFRSF11B, mRNA (LOCUS NM_002546 2354 bp mRNA linear PRI 15-MAR-2009) as deposited under accession number NM_002546 VERSION NM_002546.3 GI:148743792, gene 1..2354
   B) TNFRSF11B coding sequences (CDS) 324..1529
Figure 8 provides the nucleotide and protein sequence of stanniocalcin 1 or STC1
   A) Homo sapiens STC1, mRNA. (LOCUS NM_003155 3897 bp mRNA linear PRI 29-MAR-2009) as deposited under accession number NM_003155.2 GI:61676083
   B) STC1 coding sequences (CDS) CDS 285..1028
Figure 9 provides the nucleotide and protein sequence of PTGS2 prostaglandin-endoperoxide synthase 2 (prostaglandin G/H synthase and cyclooxygenase)
   A) Homo sapiens PTGS2 , mRNA. (LOCUS NM_000963 4507 bp mRNA linear PRI 29-MAR-2009) as deposited under accession number NM_000963 VERSION NM_000963.2 GI:223941909
   B) PTGS2 coding sequences (CDS) 138..1952
Figure 10 provides the nucleotide and protein sequence of Homo sapiens interleukin 11 (IL11
   A) Homo sapiens IL-11, mRNA. (LOCUS N_000641 2354 bp mRNA linear PRI 29-MAR-2009) as deposited under accession number N_000641 VERSION NM_000641.2 GI:24430217B)
      IL-11 coding sequences (CDS) 137..736
Figure 11 provides a schematic representation of a diabody expression cassette. The locations of promoter/operator (*p*/*o*), rbs, gene encoding Leader 1 and Leader 2 (allowing secretory production of the diabody), Tag 1 (i.e. cMyc epitope) and Tag 2 (i.e. 6HIS, if appropriate) are indicated. Amino acid sequence of the linker (L) connecting the variable domains in each antibody fragment is L = AKTTPKLGG.
Figure 12 provides a schematic representation of the assembly of the diabody expression cassette. The locations of promoter/operator (p/o), rbs, gene encoding Leader 1 (L1) and Leader 2 (L2), Tag 1 and Tag 2 are indicated. The sequence of the linker (L) connecting the variable domains in each antibody fragment was incorporated via PCR.

### References to this application.

(1) Targan SR, Hanauer SB, Van Deventer SJ et al. A short-term study of chimeric monoclonal antibody cA2 to tumor necrosis factor alpha for Crohn's disease. Crohn's Disease cA2 Study Group. N Engl J Med 1997; 337(15):1029-1035.
(2) Rutgeerts P, Feagan BG, Lichtenstein GR et al. Comparison of scheduled and episodic treatment strategies of infliximab in Crohn's disease. Gastroenterology 2004; 126(2):402-413.
(3) Present DH, Rutgeerts P, Targan S et al. Infliximab for the treatment of fistulas in patients with Crohn's disease. N Engl J Med 1999; 340(18):1398-1405.
(4) Sands BE, Anderson FH, Bernstein CN et al. Infliximab maintenance therapy for fistulizing Crohn's disease. N Engl J Med 2004; 350(9):876-885.
(5) Rutgeerts P, Sandborn WJ, Feagan BG et al. Infliximab for induction and maintenance therapy for ulcerative colitis. N Engl J Med 2005; 353(23):2462-2476.
(6) Schena M, Shalon D, Davis RW, Brown PO. Quantitative monitoring of gene expression patterns with a complementary DNA microarray. Science 1995; 270(5235):467-470.
(7) Bullinger L, Dohner K, Bair E et al. Use of gene-expression profiling to identify prognostic subclasses in adult acute myeloid leukemia. N Engl J Med 2004; 350(16):1605-1616.
(8) Heller RA, Schena M, Chai A et al. Discovery and analysis of inflammatory disease-related genes using cDNA microarrays. Proc Natl Acad Sci U S A 1997; 94(6):2150-2155.
(9) D'Haens GR, Geboes K, Peeters M, Baert F, Penninckx F, Rutgeerts P. Early lesions of recurrent Crohn's disease caused by infusion of intestinal contents in excluded ileum. Gastroenterology 1998; 114(2):262-267.
(10) Geboes K, Riddell R, Ost A, Jensfelt B, Persson T, Lofberg R. A reproducible grading scale for histological assessment of inflammation in ulcerative colitis. Gut 2000; 47(3):404-409.
(11) Gentleman RC, Carey VJ, Bates DM et al. Bioconductor: open software development for computational biology and bioinformatics. Genome Biol 2004; 5(10):R80.
(12) Irizarry RA, Hobbs B, Collin F et al. Exploration, normalization, and summaries of high density oligonucleotide array probe level data. Biostatistics 2003; 4(2):249-264.
(13) Smyth GK. Linear models and empirical bayes methods for assessing differential expression in microarray experiments. Stat Appl Genet Mol Biol 2004; 3:Article3.
(14) Tibshirani R, Hastie T, Narasimhan B, Chu G. Diagnosis of multiple cancer types by shrunken centroids of gene expression. Proc Natl Acad Sci U S A 2002; 99(10):6567-6572.
(15) Benjamini Y, Hochberg Y. Controlling the false discovery rate: a practical and powerful approach to multiple testing. Journal of the Royal Statistical Society B 1995; 85:289-300.
(16) Dennis G, Jr., Sherman BT, Hosack DA et al. DAVID: Database for Annotation, Visualization, and Integrated Discovery. Genome Biol 2003; 4(5):3.
(17) Pfaffl MW. A new mathematical model for relative quantification in real-time RT-PCR. Nucleic Acids Res 2001; 29(9):e45.
(18) Kioi M, Kawakami M, Shimamura T, Husain SR, Puri RK. Interleukin-13 receptor alpha2 chain: a potential biomarker and molecular target for ovarian cancer therapy. Cancer 2006; 107(6):1407-1418.
(19) Fichtner-Feigl S, Strober W, Kawakami K, Puri RK, Kitani A. IL-13 signaling through the IL-13 alpha2 receptor is involved in induction of TGF-betal production and fibrosis. Nat Med 2006; 12(1):99-106.
(20) Fichtner-Feigl S, Fuss IJ, Young CA et al. Induction of IL-13 triggers TGF-betal-dependent tissue fibrosis in chronic 2,4,6-trinitrobenzene sulfonic acid colitis. J Immunol 2007; 178(9):5859-5870.

| **Table 1 Baseline Characteristics of the patients** | | | |
|---|---|---|---|
| **UC patients (n=24)** | | **CD patients (n=19)** | |
| | 14/10 | | 11/8 |
| Male/Female (%) | (58.3/41.7) | Male/Female (%) | (57.9/42.1) |
| Median (IQR) age at first infusion (years) | 41.4 (32.3-50.9) | Median (IQR) age at first infusion (years) | 31.8 (23.7-47.5) |
| Median (IQR) weight at first infusion (kg) | 72.5 (67-80.3) | Median (IQR) weight at first infusion (kg) | 68 (60.5-77.5) |
| Median (IQR) duration of disease prior to first IFX (years) | 7.3 (2.7-17.1) | Median (IQR) duration of disease prior to first IFX (years) | 6.4 (3.1-20.9) |
| Extent of disease | | Extent of disease | |
| Left-sided colitis (%) | 7 (29.2) | Ileocolon (%) | 5 (26.3) |
| Pancolitis (%) | 17 (70.8) | Colon (%) | 14 (73.7) |
| Median (IQR) C-reactive protein at first IFX (mg/dL) | 4 (1.8-19.1) | Median (IQR) C-reactive protein at first IFX (mg/dL) | 10.2 (4.3-35) |
| Concomitant medication at first IFX (%) | | Concomitant medication at first IFX (%) | |
| 5-Aminosalicylates | 18 (75) | 5-Aminosalicylates | 8 (42.1) |
| Corticosteroids | 7 (29.2) | Corticosteroids | 4 (21.1) |
| Azathioprine/6-Mercaptopurine | 15 (62.5) | Azathioprine/6-Mercaptopurine | 14 (73.7) |
| Methotrexate | 0 (0) | Methotrexate | 0 (0) |
| Corticosteroids + Immunosuppressants | 3 (12.5) | Corticosteroids + Immunosuppressants | 2 (10.5) |
| Active smoking at first IFX (%) | 2 (8.3) | Active smoking at first IFX (%) | 6 (31.6) |

**Supplementary table 2. GO analyses (EASE score < 0.01 combined with counts (DE probe sets involved in the biological process) > 10) of the DE probe sets and the downregulated DE probe sets between R and NR from the LIMMA analyses in CD and IBD. The biological processes are ranked based on the increasing order of the EASE score.**

| GO analysis of DE probe sets between R and NR from LIMMA analysis in CD | | | GO analysis of DE probe sets between R and NR from LIMMA analysis in IBD | | |
|---|---|---|---|---|---|
| **Biological Process** | **Count** | **EASE score** | **Biological Process** | **Count** | **EASE score** |
| immune response | 118 | 7,04E-39 | immune response | 114 | 5,87E-38 |
| defense response | 120 | 4,42E-36 | defense response | 117 | 5,19E-36 |
| response to biotic stimulus | 122 | 1,22E-35 | response to biotic stimulus | 119 | 1,26E-35 |
| response to pest, pathogen or parasite | 80 | 6,12E-35 | response to pest, pathogen or parasite | 78 | 1,28E-34 |
| response to wounding | 67 | 9,73E-34 | response to other organism | 79 | 1,35E-33 |
| response to other organism | 80 | 4,84E-33 | response to wounding | 65 | 4,75E-33 |
| response to external stimulus | 74 | 1,73E-32 | organismal physiological process | 160 | 8,83E-32 |
| organismal physiological process | 161 | 9,29E-30 | response to external stimulus | 71 | 3,34E-31 |
| inflammatory response | 45 | 9,18E-27 | inflammatory response | 44 | 1,61E-26 |
| response to stress | 97 | 7,14E-26 | response to stress | 95 | 3,97E-26 |
| response to stimulus | 157 | 1,21E-23 | response to stimulus | 153 | 5,67E-24 |
| cell adhesion | 62 | 4,58E-17 | humoral immune response | 32 | 1,99E-18 |
| cell communication | 165 | 2,87E-15 | taxis | 26 | 1,07E-15 |
| humoral immune response | 29 | 4,86E-15 | chemotaxis | 26 | 1,07E-15 |
| signal transduction | 155 | 6,88E-15 | locomotory behavior | 26 | 2,83E-15 |
| development | 104 | 1,59E-13 | cell communication | 158 | 1,15E-14 |
| organ development | 49 | 1,76E-13 | humoral defense mechanism (sensu Vertebrata) | 24 | 2,77E-14 |
| humoral defense mechanism (sensu Vertebrata) | 23 | 6,38E-13 | signal transduction | 144 | 1,04E-12 |
| taxis | 23 | 2,14E-12 | cell adhesion | 52 | 5,13E-12 |
| chemotaxis | 23 | 2,14E-12 | behavior | 27 | 7,77E-12 |
| cell proliferation | 47 | 3,96E-12 | cell proliferation | 43 | 1,60E-10 |
| locomotory behavior | 23 | 4,82E-12 | organ development | 42 | 3,94E-10 |
| antimicrobial humoral response (sensu Vertebrata) | 18 | 1,12E-10 | antimicrobial humoral response (sensu Vertebrata) | 17 | 5,42E-10 |
| behavior | 26 | 1,19E-10 | development | 91 | 1,00E-09 |
| morphogenesis | 47 | 1,37E-10 | antimicrobial humoral response | 17 | 1,09E-09 |
| antimicrobial humoral response | 18 | 2,35E-10 | response to chemical stimulus | 34 | 2,39E-09 |
| regulation of cell proliferation | 31 | 6,26E-10 | negative regulation of biological process | 49 | 1,05E-08 |
| immune cell activation | 19 | 3,82E-10 | immune cell activation | 17 | 2,14E-08 |
| cell activation | 19 | 9,09E-10 | cell activation | 17 | 2,43E-08 |
| locomotion | 27 | 1,33E-09 | negative regulation of cellular process | 45 | 6,66E-08 |
| localization of cell | 27 | 1,33E-09 | phosphate transport | 16 | 6,69E-08 |
| cell motility | 27 | 1,33E-09 | response to abiotic stimulus | 34 | 7,40E-08 |
| angiogenesis | 15 | 2,99E-09 | cell-cell signaling | 37 | 1,74E-07 |
| blood vessel morphogenesis | 15 | 5,37E-09 | cellular defense response | 15 | 4,40E-07 |
| blood vessel development | 15 | 5,37E-09 | morphogenesis | 38 | 8,12E-07 |
| vasculature development | 15 | 5,37E-09 | inorganic anion transport | 18 | 1,43E-06 |
| organ marphogenesis | 24 | 2,18E-08 | regulation of cell proliferation | 24 | 3,37E-06 |
| positive regulation of cell proliferation | 19 | 2,45E-08 | homeostasis | 19 | 3,82E-06 |
| cell differentiation | 38 | 3,75E-08 | calcium ion homeostasis | 12 | 3,84E-06 |
| negative regulation of biological process | 49 | 4,20E-08 | regulation of body fluids | 14 | 4,08E-06 |
| response to chemical stimulus | 32 | 8,47E-08 | lymphocyte activation | 13 | 4,18E-06 |
| negative regulation of cellular process | 46 | 8,78E-08 | hemostasis | 13 | 7,74E-06 |
| response to abiotic stimulus | 33 | 6,29E-07 | innate immune response | 11 | 9,30E-06 |
| phosphate transport | 15 | 7,41E-07 | metal ion homeostasis | 14 | 9,86E-06 |
| cellular defense response | 15 | 7,41E-07 | positive regulation of cell proliferation | 15 | 1,08E-05 |
| lymphocyte activation | 14 | 1,07E-06 | wound healing | 13 | 1,10E-05 |
| positive regulation of biological process | 40 | 1,17E-06 | positive regulation of biological process | 36 | 1,71E-05 |
| regulation of body fluids | 15 | 1,18E-06 | anion transport | 18 | 1,72E-05 |
| hemostasis | 14 | 1,96E-06 | cation homeostasis | 14 | 1,85E-05 |
| inorganic anion transport | 18 | 2,58E-06 | blood coagulation | 12 | 2,19E-05 |
| cellular morphogenesis | 23 | 5,61E-06 | di-, tri-valent inorganic cation homeostasis | 13 | 2,39E-05 |
| blood coagulation | 13 | 5,81E-06 | coagulation | 12 | 2,69E-05 |
| calcium ion homeostasis | 12 | 5,82E-06 | cell ion homeostasis | 14 | 3,05E-05 |
| coagulation | 13 | 7,29E-06 | regulation of immune response | 11 | 4,36E-05 |
| death | 36 | 8,82E-06 | regulation of development | 11 | 436E-05 |
| positive regulation of physiological process | 31 | 1,27E-05 | locomotion | 19 | 4,61E-05 |
| metal ion homeostasis | 14 | 1,57E-05 | localization of cell | 19 | 4,61E-05 |
| cell surface receptor linked signal transduction | 73 | 1,60E-05 | cell motility | 19 | 4,61E-05 |
| wound healing | 13 | 1,70E-05 | skeletal development | 14 | 4,90E-05 |
| cell death | 35 | 1,89E-05 | cell differentiation | 30 | 5,34E-05 |
| apoptosis | 34 | 2,14E-05 | negative regulation of physiological process | 36 | 5,41E-05 |
| programmed cell death | 34 | 2,30E-05 | cell surface receptor linked signal transduction | 68 | 7,85E-05 |
| homeostasis | 18 | 2,61E-05 | intracellular signaling cascade | 51 | 9,96E-05 |
| cation homeostasis | 14 | 2,91E-05 | ion homeostasis | 14 | 1,01E-04 |
| anion transport | 18 | 2,98E-05 | hemopoietic or lymphoid organ development | 11 | 1,17E-04 |
| di-, tri-valent inorganic cation homeostasis | 13 | 3,66E-05 | tissue development | 16 | 1,17E-04 |
| positive regulation of cellular physiological process | 29 | 4,15E-05 | death | 32 | 1,25E-04 |
| cell ion homeostasis | 14 | 4,78E-05 | protein kinase cascade | 20 | 1,38E-04 |
| negative regulation of physiological process | 37 | 5,77E-05 | negative regulation of cellular physiological process | 34 | 1,38E-04 |
| positive regulation of cellular process | 32 | 6,04E-05 | cell homeostasis | 14 | 2,09E-04 |
| regulation of immune response | 11 | 6,28E-05 | cell death | 31 | 2,48E-04 |
| regulation of development | 11 | 6,28E-05 | apoptosis | 30 | 2,93E-04 |
| skeletal development | 14 | 7,62E-05 | programmed cell death | 30 | 3,11E-04 |
| negative regulation of cellular physiological process | 35 | 1,41E-04 | anti-apoptosis | 12 | 3,58E-04 |
| cell-cell signaling | 31 | 1,42E-04 | regulation of organismal physiological process | 13 | 4,16E-04 |
| intracellular signaling cascade | 52 | 1,54E-04 | organ morphogenesis | 16 | 5,12E-04 |
| ion homeostasis | 14 | 1,56E-04 | positive regulation of physiological process | 26 | 5,66E-04 |
| regulation of organismal physiological process | 14 | 1,67E-04 | regulation of kinase activity | 12 | 7,32E-04 |
| hemopoietic or lymphoid organ development | 11 | 1,67E-04 | regulation of protein kinase activity | 12 | 7,32E-04 |
| negative regulation of cell proliferation | 14 | 2,64E-04 | positive regulation of cellular physiological process | 25 | 7,48E-04 |
| cell homeostasis | 14 | 3,19E-04 | regulation of transferase activity | 12 | 7,79E-04 |
| cell migration | 11 | 4,56E-04 | positive regulation of cellular process | 28 | 7,86E-04 |
| regulation of apoptosis | 21 | 0,001521459 | cellular morphogenesis | 18 | 8,23E-04 |
| regulation of programmed cell death | 21 | 0,001622414 | negative regulation of apoptosis | 12 | 0,001173889 |
| protein kinase cascade | 18 | 0,001703466 | negative regulation of programmed cell death | 12 | 0,00124191 |
| tissue development | 13 | 0,005207525 | regulation of enzyme activity | 16 | 0,002122662 |
| negative regulation of apoptosis | 11 | 0,005257505 | sensory perception | 32 | 0,003376446 |
| negative regulation of programmed cell death | 11 | 0,005516967 | second-messenger-mediated signaling | 13 | 0,004398719 |
| second-messenger-mediated signaling | 13 | 0,006120657 | regulation of apoptosis | 19 | 0,005040833 |
| cell growth | 11 | 0,009884587 | regulation of programmed cell death | 19 | 0,005280844 |
| regulation of cell size | 11 | 0,009884587 | negative regulation of cell proliferation | 11 | 0,006494387 |
| immune response | 118 | 2,35E-39 | immune response | 114 | 2,51E-40 |
| defense response | 120 | 1,48E-36 | defense response | 117 | 2,10E-38 |
| response to biotic stimulus | 122 | 4,04E-36 | response to biotic stimulus | 119 | 4,73E-38 |
| response to pest, pathogen or parasite | 80 | 2,89E-35 | response to pest, pathogen or parasite | 78 | 3,04E-36 |
| response to wounding | 67 | 5,17E-34 | response to other organism | 79 | 3,15E-35 |
| response to other organism | 80 | 2,31E-33 | response to wounding | 65 | 2,07E-34 |
| response ta external stimulus | 74 | 8,73E-33 | organismal physiological process | 157 | 4,10E-33 |
| organismal physiological process | 159 | 2,97E-29 | response to external stimulus | 71 | 1,17E-32 |
| inflammatory response | 45 | 6,02E-27 | response to stress | 95 | 6,31E-28 |
| response to stress | 97 | 3,16E-26 | inflammatory response | 44 | 1,96E-27 |
| response to stimulus | 157 | 3,56E-24 | response to stimulus | 153 | 1,06E-26 |
| cell adhesion | 62 | 2,80E-17 | humoral immune response | 32 | 4,56E-19 |
| humoral immune response | 29 | 3,78E-15 | taxis | 26 | 2,94E-16 |
| cell communication | 163 | 5,40E-15 | chemotaxis | 26 | 2,94E-16 |
| signal transduction | 153 | 1,40E-14 | locomotory behavior | 26 | 8,79E-16 |
| development | 104 | 8,01E-14 | cell communication | 154 | 1,91E-15 |
| organ development | 49 | 1,19E-13 | humoral defense mechanism (sensu Vertebrata) | 24 | 9,33E-15 |
| humoral defense mechanism (sensu Vertebrata) | 23 | 5,21E-13 | signal transduction | 141 | 1,28E-13 |
| taxis | 23 | 1,75E-12 | behavior | 27 | 2,44E-12 |
| chemotaxis | 23 | 1,75E-12 | cell adhesion | 51 | 2,58E-12 |
| cell proliferation | 47 | 2,70E-12 | development | 91 | 5,92E-11 |
| locomotory behavior | 23 | 3,95E-12 | organ development | 42 | 8,25E-11 |
| antimicrobial humoral response (sensu Vertebrata) | 18 | 9,55E-11 | cell proliferation | 42 | 1,15E-10 |
| behavior | 26 | 9,63E-11 | antimicrobial humoral response (sensu Vertebrata) | 17 | 2,57E-10 |
| morphogenesis | 47 | 9,74E-11 | antimicrobial humoral response | 17 | 5,17E-10 |
| antimicrobial humoral response | 18 | 2,01E-10 | response to chemical stimulus | 34 | 6,51E-10 |
| regulation of cell proliferation | 31 | 4,93E-10 | negative regulation of biological process | 48 | 5,72E-09 |
| immune cell activation | 19 | 6,66E-10 | immune cell activation | 17 | 1,04E-0S |
| cell activation | 19 | 7,74E-10 | cell activation | 17 | 1,19E-08 |
| locomotion | 27 | 1,08E-09 | negative regulation of cellular process | 45 | 1,47E-48 |
| localization of cell | 27 | 1,08E-09 | response to abiotic stimulus | 34 | 2,17E-08 |
| cell motility | 27 | 1,08E-09 | phosphate transport | 16 | 3,42E-08 |
| angiogenesis | 15 | 2,63E-09 | cell-cell signaling | 36 | 1,49E-07 |
| blood vessel morphogenesis | 15 | 4,72E-09 | morphogenesis | 38 | 2,30E-07 |
| blood vessel development | 15 | 4,72E-09 | cellular defense response | 15 | 2,37E-07 |
| vasculature development | 15 | 4,72E-09 | regulation of cell proliferation | 24 | 1,42E-05 |
| organ morphogenesis | 24 | 1,82E-08 | regulation of body fluids | 14 | 2,33E-06 |
| positive regulation of cell proliferation | 19 | 2,10E-08 | calcium ion homeostasis | 12 | 2,35E-06 |
| cell differentiation | 38 | 2,89E-08 | lymphocyte activation | 13 | 2,47E-06 |
| negative regulation of biological process | 49 | 3,05E-08 | hemostasis | 13 | 4,32E-06 |
| negative regulation of cellular process | 46 | 6,50E-08 | positive regulation of biological process | 36 | 5,64E-06 |
| response to chemical stimulus | 32 | 6,85E-08 | innate immune response | 11 | 5,95E-06 |
| response to abiotic stimulus | 33 | 5,02E-07 | positive regulation of cell proliferation | 15 | 6,07E-06 |
| phosphate transport | 15 | 6,57E-07 | wound healing | 13 | 6,59E-06 |
| cellular defense response | 15 | 6,57E-07 | homeostasis | 18 | 7,78E-06 |
| positive regulation of biological process | 40 | 9,15E-07 | blood coagulation | 12 | 1,37E-05 |
| lymphocyte activation | 14 | 9,53E-07 | di-, tri-valent inorganic cation homeostasis | 13 | 1,44E-05 |
| regulation of body fluids | 15 | 1,04E-06 | cell surface receptor linked signal transduction | 68 | 1,48E-05 |
| hemostasis | 14 | 1,75E-06 | inorganic anion transport | 16 | 1,48E-05 |
| cellular morphogenesis | 23 | 4,82E-06 | coagulation | 12 | 1,68E-05 |
| blood coagulation | 13 | 5,24E-06 | negative regulation of physiological process | 36 | 1,87E-05 |
| calcium ion homeostasis | 12 | 5,29E-06 | cell differentiation | 30 | 2,11E-05 |
| coagulation | 13 | 6,58E-06 | locomotion | 19 | 2,35E-05 |
| death | 36 | 7,09E-06 | localization of cell | 19 | 2,35E-05 |
| inorganic anion transport | 17 | 9,76E-06 | cell motility | 19 | 2,35E-05 |
| positive regulation of physiological process | 31 | 1,04E-05 | intracellular signaling cascade | 51 | 2,61E-05 |
| cell surface receptor linked signal transduction | 73 | 1,13E-05 | regulation of immune response | 11 | 2,83E-05 |
| cell death | 35 | 1,53E-05 | regulation of development | 11 | 2,83E-05 |
| wound healing | 13 | 1,54E-05 | skeletal development | 14 | 2,89E-05 |
| apoptosis | 34 | 1,73E-05 | metal ion homeostasis | 13 | 2,94E-05 |
| programmed cell death | 34 | 1,86E-05 | death | 32 | 4,80E-05 |
| di-, tri-valent inorganic cation homeostasis | 13 | 3,32E-05 | negative regulation of cellular physiological process | 34 | 5,19E-05 |
| positive regulation of cellular physiological process | 29 | 3,47E-05 | cation homeostasis | 13 | 5,22E-05 |
| negative regulation of physiological process | 37 | 4,75E-05 | tissue development | 16 | 6,65E-05 |
| positive regulation of cellular process | 32 | 5,00E-05 | protein kinase cascade | 20 | 7,02E-05 |
| regulation of immune response | 11 | 5,77E-05 | hemopoietic or lymphoid organ development | 11 | 7,69E-05 |
| regulation of development | 11 | 5,77E-05 | cell ion homeostasis | 13 | 8,25E-05 |
| metal ion homeostasis | 13 | 6,66E-05 | cell death | 31 | 1,01E-04 |
| skeletal development | 14 | 6,89E-05 | apoptosis | 30 | 1,23E-04 |
| homeostasis | 17 | 8,32E-05 | anion transport | 16 | 1,25E-04 |
| anion transport | 17 | 9,36E-05 | programmed cell death | 30 | 1,31E-04 |
| cation homeostasis | 13 | 1,17E-04 | anti-apoptosis | 12 | 2,31E-04 |
| negative regulation of cellular physiological process | 35 | 1,17E-04 | ion homeostasis | 13 | 2,46E-04 |
| cell-cell signaling | 31 | 1,19E-04 | regulation of organismal physiological process | 13 | 2,62E-04 |
| intracellular signaling cascade | 52 | 1,20E-04 | positive regulation of physiological process | 26 | 2,65E-04 |
| regulation of organismal physiological process | 14 | 1,51E-04 | organ morphogenesis | 16 | 3,02E-04 |
| hemopoietic or lymphoid organ development | 11 | 1,54E-04 | positive regulation of cellular physiological process | 25 | 3,51E-04 |
| cell ion homeostasis | 13 | 1,82E-04 | positive regulation of cellular process | 28 | 3,59E-04 |
| negative regulation of cell proliferation | 14 | 2,40E-04 | cellular morphogenesis | 18 | 4,62E-04 |
| cell migration | 11 | 4,22E-04 | cell homeostasis | 13 | 4,78E-04 |
| ion homeostasis | 13 | 5,28E-04 | negative regulation of apoptosis | 12 | 7,76E-04 |
| cell homeostasis | 13 | 0,00100076 | negative regulation of programmed cell death | 12 | 8,22E-04 |
| regulation of apoptosis | 21 | 0,001371422 | sensory perception | 32 | 0,001542761 |
| regulation of programmed cell death | 21 | 0,001460683 | regulation of kinase activity | 11 | 0,001773554 |
| protein kinase cascade | 18 | 0,001541729 | regulation of protein kinase activity | 11 | 0,001773554 |
| tissue development | 13 | 0,004845121 | regulation of transferase activity | 11 | 0,001873001 |
| negative regulation of apoptosis | 11 | 0,004904162 | second-messenger-mediated signaling | 13 | 0,002922318 |
| negative regulation of programmed cell death | 11 | 0,005147377 | regulation of apoptosis | 19 | 0,002964354 |
| second-messenger-mediated signaling | 13 | 0,005699655 | regulation of programmed cell death | 19 | 0,003115078 |
| cell growth | 11 | 0,009249997 | regulation of enzyme activity | 15 | 0,003480013 |
| regulation of cell size | 11 | 0,009249997 | negative regulation of cell proliferation | 11 | 0,00457427 |
| | | | growth | 12 | 0,008354378 |

**Supplementary table 3. The subset of probe sets, identified by PAM analysis in UC, CD, and IBD. The scores indicate whether the probe set is up- or downregulated in the two classes of samples. The probe sets are ranked based on the decreased predictive value.**

| **UC (R=8, NR=16)** | **Probe Set ID** | **Gene Symbol** | **Gene Title** | **NR-score** | **R-score** |
|---|---|---|---|---|---|
| 1 | 206172_at | IL13RA2 | interleukin 13 receptor, alpha 2 | 0,2108 | -0,4215 |
| 2 | 205680_at | MMP10 | matrix metallopeptidase 10 (stromelysin 2) | 0,0749 | -0,1497 |
| 3 | 206336_at | CXCL6 | chemokine (C-X-C motif) ligand 6 (granulocyte chemotactic protein 2) | 0,0687 | -0,1374 |
| 4 | 231227_at | --- | Transcribed locus | 0,0629 | -0,1258 |
| 5 | 206953_s_at | LPHN2 | latrophilin 2 | 0,0614 | -0,1228 |
| 6 | 227140_at | --- | CDNA FLJ11041 fis, clone PLACE1004405 | 0,0613 | -0,1226 |
| 7 | 205990_s_at | WNT5A | wingless-type MMTV integration site family, member 5A | 0,0574 | -0,1147 |
| 8 | 206924_at | IL11 | interleukin 11 | 0,0556 | -0,1112 |
| 9 | 212526_at | SPG20 | spastic paraplegia 20 (Troyer syndrome) | 0,0412 | -0,0824 |
| 10 | 213338_at | TMEM158 | transmembrane protein 158 | 0,0382 | -0,0764 |
| 11 | 211959_at | IGFBP5 | insulin-like growth factor binding protein 5 | 0,038 | -0,0759 |
| 12 | 204597_x_at | STC1 | stanniocalcin 1 | 0,0373 | -0,0747 |
| 13 | 204933_s_at | TNFRSF 11 B | tumor necrosis factor receptor superfamily, member 11b (osteoprotegerin) | 0,0357 | -0,0714 |
| 14 | 204222_s_at | GLIPR1 | GLI pathogenesis-related 1 (glioma) | 0,0332 | -0,0664 |
| 15 | 227361_at | HS3ST3B1 | heparan sulfate (glucosamine) 3-O-sulfotransferase 3B1 1 | 0,029 | -0,058 |
| 16 | 203424_s_at | IGFBP5 | insulin-like growth factor binding protein 5 | 0,0228 | -0,0457 |
| 17 | 211671_s_at | NR3C1 | nuclear receptor subfamily 3, group C, member 1 (glucocorticoid receptor) | 0,0221 | -0,0441 |
| 18 | 201645_at | TNC | tenascin C (hexabrachion) | 0,0211 | -0,0422 |
| 19 | 230746_s_at | STC1 | Stanniocalcin 1 | 0,0187 | -0,0374 |
| 20 | 228128_x_at | PAPPA | pregnancy-associated plasma protein A, pappalysin 1 | 0,0187 | -0,0374 |
| 21 | 203603_s_at | ZEB2 | zinc finger E-box binding homeobox 2 | 0,0185 | -0,0369 |
| 22 | 209795_at | CD69 | CD69 molecule | 0,018 | -0,0359 |
| 23 | 206623_at | PDE6A | phosphodiesterase 6A, cGMP-specific, rod, alpha | -0,0166 | 0,0332 |
| 24 | 212977_at | CXCR7 | chemokine (C-X-C motif) receptor 7 | 0,016 | -0,0319 |
| 25 | 203887_s_at | THBD | thrombomodulin | 0,0153 | -0,0306 |
| 26 | 202422_s_at | ACSL4 | acyl-CoA synthetase long-chain family member 4 | 0,0121 | -0,0243 |
| 27 | 203680_at | PRKAR2B | protein kinase, cAMP-dependent, regulatory, type II, beta | 0,0118 | -0,0236 |
| 28 | 1555638_a_at | SAMSN1 | SAM domain, SH3 domain and nuclear localization signals 1 | 0,011 | -0,0219 |
| 29 | 226001_at | KLHL5 | kelch-like 5 (Drosophila) | 0,0084 | -0,0169 |
| 30 | 205207_at | IL6 | interleukin 6 (interferon, beta 2) | 0,0064 | -0,0127 |
| 31 | 207610_s_at | EMR2 | egf-like module containing, mucin-like, hormone receptor-like 2 | 0,0062 | -0,0125 |
| 32 | 205443_at | SNAPC1 | small nuclear RNA activating complex, polypeptide 1, 43kDa | 0,0059 | -0,0118 |
| 33 | 205828_at | MMP3 | matrix metallopeptidase 3 (stromelysin 1, progelatinase) | 0,0046 | -0,0092 |
| 34 | 1555229_a_at | C1S | complement component 1, s subcomponent | 0,0041 | -0,0083 |
| 35 | 204932_at | TNFRSF11B | tumor necrosis factor receptor superfamily, member 11b (osteoprotegerin) | 0,0036 | -0,0072 |
| 36 | 214247_s_at | DKK3 | dickkopf homolog 3 (Xenopus laevis) | 0,0029 | -0,0058 |
| 37 | 209960 at | HGF | hepatocyte growth factor (hepapoietin A; scatter factor) | 0,0026 | -0,0053 |
| | | | | | |

| **CD (R=12, NR=7)** | **Probe Set ID** | **Gene Symbol** | **Gene Title** | **NR-score** | **R-score** |
|---|---|---|---|---|---|
| 1 | 206025_s_at | TNFAIP6 | tumor necrosis factor, alpha-induced protein 6 | 0,7572 | -0,4417 |
| 2 | 206026_s_at | TNFAIP6 | tumor necrosis factor, alpha-induced protein 6 | 0,4848 | -0,2828 |
| 3 | 206924_at | IL11 | interleukin 11 | 0,4385 | -0,2558 |
| 4 | 213524_s_at | G0S2 | G0/G1 switch 2 | 0,3358 | -0,1959 |
| 5 | 214370_at | S100A8 | S100 calcium binding protein A8 | 0,2959 | -0,1726 |
| 6 | 205863_at | S100A12 | S100 calcium binding protein A12 | 0,2852 | -0,1664 |
| 7 | 203535_at | S100A9 | S100 calcium binding protein A9 | 0,2219 | -0,1294 |
| 8 | 205681_at | BCL2A1 | BCL2-related protein A1 | 0,1908 | -0,1113 |
| 9 | 232629_at | PROK2 | prokineticin 2 | 0,1904 | -0,1111 |
| 10 | 205207_at | IL6 | interleukin 6 (interferon, beta 2) | 0,1646 | -0,096 |
| 11 | 204959_at | MNDA | myeloid cell nuclear differentiation antigen | 0,1167 | -0,0681 |
| 12 | 222088_s_at | SLC2A14 /// SLC2A3 | solute carrier family 2 (facilitated glucose transporter), member 3 | 0,108 | -0,063 |
| | | | /// solute carrier family 2 (facilitated glucose transporter), member | | |
| | | | 14 | | |
| 13 | 206172_at | IL13RA2 | interleukin 13 receptor, alpha 2 | 0,1053 | -0,0614 |
| 14 | 202917_s_at | S100A8 | S100 calcium binding protein A8 | 0,1015 | -0,0592 |
| 15 | 229947_at | PI15 | peptidase inhibitor 15 | 0,0805 | -0,047 |
| 16 | 205119_s_at | FPR1 | formyl peptide receptor 1 | 0,0775 | -0,0452 |
| 17 | 1554997_a_at | PTGS2 | prostaglandin-endoperoxide synthase 2 | 0,0445 | -0,0259 |
| | | | (prostaglandin G/H synthase and cyclooxygenase) | | |
| 18 19 | 202499_s_at 205568_at | SLC2A3 AQP9 | solute carrier family 2 (facilitated glucose transporter), member 3 aquaporin 9 | 0,0318 0,0252 | -0,0185 -0,0147 |
| 20 | 229723_at | TAGAP | T-cell activation GTPase activating protein | 0,024 | -0,014 |
| 21 | 209949 at | NCF2 | neutrophil cytosolic factor 2 (65kDa, chronic granulomatous disease, autosomal 2) | 0,016 | -0,0094 |
| | | | | | |

| **IBD (R=20, NR=23)** | **Probe Set ID** | **Gene Symbol** | **Gene Title** | **NR-score** | **R-score** |
|---|---|---|---|---|---|
| 1 | 206172_at | IL13RA2 | interleukin 13 receptor, alpha 2 | 0,1886 | -0,2169 |
| 2 | 206924 at | IL11 | interleukin 11 | 0,016 | -0,0184 |

## Claims

1. An in vitro method of diagnosing for predicting if a subject suffering of an inflammatory bowel disease will respond to an anti-TNFα therapy, such method comprises: (a) analyzing the level of STC1 and IL-13Rα2 mRNA expression in a biological sample isolated from said subject, and (b) compare said level of expression with the STC1 and IL-13Rα2 mRNA expression in a control sample; whereby a decreased level of STC1 and IL-13Rα2 mRNA expression relative to a control sample is an indication of response to anti-TNFα therapy or a propensity thereto; and wherein an increased level of STC1 and IL-13Rα2 mRNA expression relative to a control sample is an indication of non-response to anti-TNFα therapy or a propensity thereto.

2. The in vitro method of diagnosing of claim 1 whereby the inflammatory bowel disease is Crohn's disease.

3. The in vitro method of diagnosing of any of the claims 1-2 wherein the anti-TNFα therapy is an anti-TNFα antibody therapy such as a therapy with an anti-TNFα therapeutic antibody for instance an antibody that blocks the action of TNFα by preventing it from binding to its receptor in the cell such as infliximab, adalimumab or etanercept.

4. The in vitro method of diagnosis of any of the previous claims, to predict the responding or non responding of a subject on an anti-TNFα treatment of an inflammatory bowel disease, or a propensity thereto in a subject, said method (a) obtaining an expression profile in a biological sample isolated from said subject, wherein said expression profile consists of the analysis of the level of STC1 and IL-13Ra2 mRNA expression in combination with the mRNA expression level of at least one, two, or three, of the gene cluster of gene(s) selected from the group consisting of TNFRS11b, PTGS2, and IL-11; and (b) comparing said obtained expression profile to a reference expression profile to determine whether said sample is from subject having a IBD phenotype or a propensity thereto.

5. The in vitro method of diagnosis of claim 4; the expression profile consisting of any one of the following combinations; STC1 and IL-13Rα2; STC1 and IL-13Rα2 and PTGS2; STC1 and IL-13Rα2 and TNFRSF11B; STC1 and IL-13Rα2 and IL-11; STC1 and IL-13Rα2 and TNFRSF11B and PTGS2; STC1 and IL-13Rα2 and IL-11 and PTGS2; STC1 and IL-13Rα2 and TNFRSF11B and IL-11.

6. The in-vitro method according to any one of the previous claims comprising the detection of the level of the nucleic acids carried out using at least one binding agent specifically binding to the nucleic acids to be detected; in particular said binding agent being detectably labelled; more in particular with a label selected from the group consisting of a radioisotope, a bioluminescent compound, a chemiluminescent compound, a fluorescent compound, a metal chelate, biotin, digoxygenin and an enzyme.

7. The in-vitro method according to any of the previous claims, at least one binding agent being a nucleic acid hybridising to a nucleic acid is used for the detection of the marker molecules, in particular for the detection of IL-13Rα2, TNFRS11b, STC1 PTGS2, and IL-11 expression

8. A diagnostic test kit for use in diagnosing a subject for responsiveness on a anti-TNFα treatment of inflammatory bowel disease (IBD) such as Crohn's disease (CD), or for use in monitoring the effectiveness of therapy of inflammatory bowel disease (IBD) such as Crohn's disease (CD) in patients receiving an anti-TNFα therapy comprising: a) a nucleic acid encoding the STC1 protein; b) reagents useful for monitoring the expression level of the one or more nucleic acids of step a); c) instructions for use of the kit.

9. The diagnostic test kit of claim 8, for use in diagnosing a subject for responsiveness on a anti-TNFα treatment of inflammatory bowel disease (IBD) such as Crohn's disease (CD), or for use in monitoring the effectiveness of therapy of inflammatory bowel disease (IBD) in patients receiving an anti-TNFα therapy comprising: a) nucleic acids encoding the IL-13Rα2, TNFRS11b, STC1 PTGS2, and IL-11 protein; b) reagents useful for monitoring the expression level of the one or more nucleic acids of step a); c) instructions for use of the kit.

## Patentansprüche

1. Eine in-vitro-Methode zur diagnostischen Voraussage der voraussichtlichen Reaktion eines an einer chronisch entzündlichen Darmerkrankung leidenden Patienten auf eine Anti-TNFα-Therapie, wobei die betreffende Methode aus den folgenden Elementen besteht: (a) Analyse der STC1- und IL-13Rα2-mRNA-Expression in einer dem besagten Patienten entnommenen, isolierten biologischen Probe, und (b) Vergleich der besagten Expression mit der STC1- und IL-13Rα2-mRNA-Expression einer Kontrollprobe, wobei eine STC1- und IL-13Rα2-mRNA-Expression der dem Patienten entnommenen Probe, deren Werte unter denen der Kontrollprobe liegen, als Anzeichen für eine voraussichtliche Reaktion des betreffenden Patienten auf eine Anti-TNFα-Therapie bzw. eine entsprechende Neigung zu interpretieren ist und wobei eine STC1-und IL-13Rα2-mRNA-Expression der dem Patienten entnommenen Probe, deren Werte über denen der Kontrollprobe liegen, als Anzeichen für eine voraussichtliche Nicht-Reaktion des betreffenden Patienten auf eine Anti-TNFα-Therapie bzw. eine entsprechende Neigung zu interpretieren ist.

2. Die in-vitro-Diagnosemethode gemäß Anspruch 1, wobei es sich bei der chronisch entzündlichen Darmerkrankung um Morbus Crohn handelt.

3. Die in-vitro-Diagnosemethode gemäß einem der Ansprüche 1 oder 2, wobei es sich bei der Anti-TNFα-Therapie um eine Anti-TNFα-Antikörpertherapie wie etwa eine Therapie mit einem therapeutischen Anti-TNFα-Antikörper handelt, z.B. mit einem Antikörper wie etwa Infliximab, Adalimumab oder Etanercept, der die TNFα-Wirkung durch Verhinderung einer Bindung an den betreffenden Rezeptoren in der Zelle blockiert.

4. Die in-vitro-Diagnosemethode zur diagnostischen Voraussage der voraussichtlichen Reaktion bzw. Nicht-Reaktion (oder der einschlägigen Neigung) eines Patienten auf eine Anti-TNFα-Therapie gegen eine chronisch entzündliche Darmerkrankung gemäß den vorhergehenden Ansprüchen, wobei die besagte Methode die folgenden Elemente beinhaltet: (a) die Erstellung eines Expressionsprofils in einer dem besagten Patienten entnommenen, isolierten Probe, das aus der Analyse der STC1- und IL-13Rα2-mRNA-Expression in Kombination mit der mRNA-Expression von wenigstens einem, zweien oder dreien in einem Gencluster vereinten Gene aus der Gruppe TNFRS11b, PTGS2 und IL-11 besteht; und (b) der Vergleich zwischen dem besagten Expressionsprofil und einem Referenzexpressionsprofil, wobei anhand dieses Vergleichs ermittelt werden soll, ob der Patient, dem die besagte Probe entnommen wurde, ein aIBD-Erscheinungsbild bzw. eine entsprechende Neigung besitzt.

5. Die in-vitro-Diagnosemethode gemäß Anspruch 4, wobei das Expressionsprofil aus einer der folgenden Kombinationen besteht: STC1 und IL-13Rα2; STC1 und IL-13Rα2 und PTGS2; STC1 und IL-13Rα2 und TNFRSF11B; STC1 und IL-13Rα2 und IL-11; STC1 und IL-13Rα2 und TNFRSF11B und PTGS2; STC1 und IL-13Rα2 und IL-11 und PTGS2; STC1 und IL-13Rα2 und TNFRSF11B und IL-11.

6. Die in-vitro-Methode gemäß einem der vorhergehenden Ansprüche einschließlich eines Vorgangs zur Bestimmung der Menge von Nukleinsäuren, wobei mindestens ein Bindemittel eingesetzt wird, das sich spezifisch an die zu bestimmenden Nukleinsäuren bindet; insbesondere mit einer erkennbaren Kennzeichnung des besagten Bindemittels; und hier insbesondere mit einer Kennzeichnung, bei der es sich um ein Radioisotop, eine biolumineszente Verbindung, eine chemilumineszendierende Verbindung, eine fluoreszendierende Verbindung, ein Metallchelat, Biotin, Digoxigenin und ein Enzym handelt.

7. Die in-vitro-Methode gemäß einem der vorhergehenden Ansprüche, wobei mindestens ein - aus einer zu einer Nukleinsäure hybridisierenden Nukleinsäure bestehenden - Bindemittel zur Bestimmung der Markermoleküle verwendet wird, insbesondere zur Bestimmung der Expression von IL-13Rα2, TNFRS11b, STC1 PTGS2 und IL-11.

8. Ein diagnostisches Testkit zur Diagnose der Ansprechbarkeit von Patienten auf Anti-TNFα-Therapien gegen chronisch entzündliche Darmerkrankungen wie Morbus Crohn oder zur Überprüfung der Wirksamkeit von Anti-TNFα-Therapien bei der Behandlung von Patienten mit chronisch entzündlichen Darmerkrankungen wie Morbus Crohn, wobei das besagte Testkit aus den folgenden Elementen besteht: a) eine das STC1-Protein kodierende Nukleinsäure; b) Reagenzien, die sich zur Beobachtung des Expressionsniveaus von einer oder mehreren Nukleinsäuren gemäß Schritt a) eignen; c) eine Anleitung zur Benutzung des Testkits.

9. Das diagnostische Testkit von Anspruch 8 zur Diagnose der Ansprechbarkeit von Patienten auf Anti-TNFα-Therapien gegen chronisch entzündliche Darmerkrankungen wie Morbus Crohn oder zur Überprüfung der Wirksamkeit von Anti-TNFα-Therapien bei der Behandlung von Patienten mit chronisch entzündlichen Darmerkrankungen, wobei das besagte Testkit aus den folgenden Elementen besteht: a) Nukleinsäuren, welche die Proteine IL-13Rα2, TNFRS11b, STC1 PTGS2 und IL-11 kodieren; b) Reagenzien, die sich zur Beobachtung des Expressionsniveaus von einer oder mehreren Nukleinsäuren gemäß Schritt a) eignen; c) eine Anleitung zur Benutzung des Testkits.

## Revendications

1. Une méthode in vitro de diagnostique pour prévoir si un sujet souffre d'une maladie intestinale inflammatoire et s'il répondra à la thérapie anti-TNFα, cette méthode comprenant : (a) l'analyse du niveau d'expression de STC1 et de IL-13Rα2 mRNA dans un échantillon biologique isolé dudit sujet , et (b) comparer lesdits niveaux d'expression avec l'expression du STC1 et du IL-13Rα2 mRNA dans un échantillon témoin ; ou un niveau réduit d'expression du STC1 et d'expression IL-13Rα2 mRNA relatif à l'échantillon témoin est une indication de réponse à la thérapie anti-TNFα au à une proportion à celle-ci ; et si un niveau élevé d'expression de STC1 et d'expression de IL-13Rα2 mRNA relatif à l'échantillon témoin est une indication d'absence de réponse à la thérapie anti-TNFα ou à une propension à celle-ci.

2. La méthode in vitro de diagnostique de la revendication 1 ou la maladie intestinale inflammatoire est la maladie de Crohn.

3. La méthode in vitro de diagnostique de l'une des revendications 1 - 2 où la thérapie anti-TNFα est une thérapie anticorps anti-TNFα comme une thérapie avec un anticorps thérapeutique anti-TNFα, par exemple un anticorps qui bloque l'action du TNFα en empêchant de se lier à son récepteur dans la cellule comme l'infliximab, l'adalimumab ou l'etanercept.

4. La méthode in vitro de diagnostique de l'une des revendications précédentes, prévoyant la réponse ou la non-réponse de sujet au traitement anti-TNFα d'une maladie intestinale inflammatoire, ou une propension chez le sujet, ladite méthode (a) obtenant un profil expression dans un échantillon biologique isolé dudit sujet , ou le profil de ladite expression consiste en l'analyse du niveau d'expression de STC1 et de IL-13Rα2 A en combinaison avec le niveau d'expression de mRNA d'au moins un, deux ou trois groupes de gène (s) sélectionné(s) dans le groupe consistant en TNFRS11b, PTGS2, et en IL-11; et (b) en comparant ledit profil d'expression obtenu dans un profil d'expression de référence afin de déterminer si l'échantillon provient d'un sujet ayant un phénotype IBD ou s'il a une propension .

5. La méthode in vitro de diagnostique de la revendication 4 ; le profil expression consistant en une des combinaisons suivantes ; STC1 et IL-13Rα2; STC1 et IL-13Rα2 et PTGS2; STC1 et IL-13Rα2 et TNFRSF11B; STC1 et IL-13Rα2 et IL-11; STC1 et IL-13Rα2 et TNFRSF11B et PTGS2; STC1 et IL-13Rα2 et IL-11 et PTGS2; STC1 et IL-13Rα2 et TNFRSF11B et IL-11.

6. La méthode in vitro selon l'une des revendications précédentes comprenant la détection du niveau des acides nucléiques existants au moins dans un agent de liaison spécialement lié aux acides nucléiques à détecter ; en particulier ledit agent de liaison étant étiqueté de manière détectable ; plus en particulier avec une étiquette sélectionnée dans le groupe consistant en un radio-isotope, un composant bioluminescent , un composant chimiluminescent, un composant fluorescent, un chélate métallique, de abiotine, dela dioxygénine et un enzyme.

7. La méthode in vitro selon l'une des revendications précédentes, au moins un agent de liaison étant une hybridation de l'acide nucléique vers un acide nucléique utilisé pour la détection des molécules marqueurs, en particulier pour la détection de IL-13Ra2, TNFRS11b, STC1 PTGS2, et l'expression de IL-11

8. Un test de diagnostique pour établir un diagnostique chez un sujet concernant la réactivité dans un traitement anti-TNFα dans une maladie intestinale inflammatoire (IBD) comme la maladie de Crohn (CD), ou pour l'utilisation dans le contrôle de l'efficacité de la thérapie de la maladie intestinale inflammatoire (IBD) comme la maladie de Crohn (CD) chez les patients recevant une thérapie anti-TNFα comprenant a) un encodage d'acide nucléique dans la protéine STC1 ; b) les réactifs utiles pour le contrôle du niveau d'expression de l'un ou de plusieurs acides nucléiques dans l'étape a); c) instructions d'usage du kit.

9. Le test diagnostique de la revendication 8, dans l'utilisation d'un diagnostique chez un sujet concernant la capacité de réponse dans un traitement anti-TNFα dans la maladie intestinale inflammatoire (IBD) comme la maladie de Crohn (CD), ou dans l'utilisation du contrôle de l'efficacité de la thérapie de la maladie intestinale inflammatoire (IBD) chez des patients recevant une thérapie anti-TNFα comprenant : a) l'encodage des acides nucléiques du IL-13Rα2, TNFRS11b, STC1 PTGS2, et de la protéine IL-11 ; b) les réactifs utiles dans le contrôle du niveau d'expression de l'un ou de plusieurs acides nucléiques de l'étape a); c) les instructions pour l'utilisation du kit.


### SEQUENCE LISTING

<110> Katholieke Universiteit Leuven
<120> MUCOSAL GENE SIGNATURES
<130> LRD-PCT-2008025
<140> NOT YET KNOWN
   <141> 2009-03-28
<150> US 61/072,200
   <151> 2008-03-28
<160> 10
<170> PatentIn version 3.4
<210> 1
   <211> 1376
   <212> DNA
   <213> Homo sapiens
<400> 1
   gtaagaacac tctcgtgagt ctaacggtct tccggatgaa ggctatttga agtcgccata      60
   acctggtcag aagtgtgcct gtcggcgggg agagaggcaa tatcaaggtt ttaaatctcg     120
   gagaaatggc tttcgtttgc ttggctatcg gatgcttata tacctttctg ataagcacaa      180
   catttggctg tacttcatct tcagacaccg agataaaagt taaccctcct caggattttg     240
   agatagtgga tcccggatac ttaggttatc tctatttgca atggcaaccc ccactgtctc     300
   tggatcattt taaggaatgc acagtggaat atgaactaaa ataccgaaac attggtagtg     360
   aaacatggaa gaccatcatt actaagaatc tacattacaa agatgggttt gatcttaaca     420
   agggcattga agcgaagata cacacgcttt taccatggca atgcacaaat ggatcagaag     480
   ttcaaagttc ctgggcagaa actacttatt ggatatcacc acaaggaatt ccagaaacta     540
   aagttcagga tatggattgc gtatattaca attggcaata tttactctgt tcttggaaac     600
   ctggcatagg tgtacttctt gataccaatt acaacttgtt ttactggtat gagggcttgg     660
   atcatgcatt acagtgtgtt gattacatca aggctgatgg acaaaatata ggatgcagat     720
   ttccctattt ggaggcatca gactataaag atttctatat ttgtgttaat ggatcatcag     780
   agaacaagcc tatcagatcc agttatttca cttttcagct tcaaaatata gttaaacctt     840
   tgccgccagt ctatcttact tttactcggg agagttcatg tgaaattaag ctgaaatgga     900
   gcataccttt gggacctatt ccagcaaggt gttttgatta tgaaattgag atcagagaag     960
   atgatactac cttggtgact gctacagttg aaaatgaaac atacaccttg aaaacaacaa    1020
   atgaaacccg acaattatgc tttgtagtaa gaagcaaagt gaatatttat tgctcagatg    1080
   acggaatttg gagtgagtgg agtgataaac aatgctggga aggtgaagac ctatcgaaga    1140
   aaactttgct acgtttctgg ctaccatttg gtttcatctt aatattagtt atatttgtaa    1200
   ccggtctgct tttgcgtaag ccaaacacct acccaaaaat gattccagaa tttttctgtg    1260
   atacatgaag actttccata tcaagagaca tggtattgac tcaacagttt ccagtcatgg    1320
   ccaaatgttc aatatgagtc tcaataaact gaatttttct tgcgaatgtt gaaaaa        1376
<210> 2
   <211> 380
   <212> PRT
   <213> Homo Sapiens
<400> 2
   Met Ala Phe Val Cys Leu Ala Ile Gly Cys Leu Tyr Thr Phe Leu Ile
   1               5                   10                  15
   Ser Thr Thr Phe Gly Cys Thr Ser Ser Ser Asp Thr Glu Ile Lys Val
               20                  25                  30
   Asn Pro Pro Gln Asp Phe Glu Ile Val Asp Pro Gly Tyr Leu Gly Tyr
           35                  40                  45
   Leu Tyr Leu Gln Trp Gln Pro Pro Leu Ser Leu Asp His Phe Lys Glu
       50                  55                  60
   Cys Thr Val Glu Tyr Glu Leu Lys Tyr Arg Asn Ile Gly Ser Glu Thr
   65                  70                  75                  80
   Trp Lys Thr Ile Ile Thr Lys Asn Leu His Tyr Lys Asp Gly Phe Asp
                   85                  90                  95
   Leu Asn Lys Gly Ile Glu Ala Lys Ile His Thr Leu Leu Pro Trp Gln
               100                 105                 110
   Cys Thr Asn Gly Ser Glu Val Gln Ser Ser Trp Ala Glu Thr Thr Tyr
           115                 120                 125
   Trp Ile Ser Pro Gln Gly Ile Pro Glu Thr Lys Val Gln Asp Met Asp
       130                 135                 140
   Cys Val Tyr Tyr Asn Trp Gln Tyr Leu Leu Cys Ser Trp Lys Pro Gly
   145                 150                 155                 160
   Ile Gly Val Leu Leu Asp Thr Asn Tyr Asn Leu Phe Tyr Trp Tyr Glu
                   165                 170                 175
   Gly Leu Asp His Ala Leu Gln Cys Val Asp Tyr Ile Lys Ala Asp Gly
               180                 185                 190
   Gln Asn Ile Gly Cys Arg Phe Pro Tyr Leu Glu Ala Ser Asp Tyr Lys
           195                 200                 205
   Asp Phe Tyr Ile Cys Val Asn Gly Ser Ser Glu Asn Lys Pro Ile Arg
       210                 215                 220
   Ser Ser Tyr Phe Thr Phe Gln Leu Gln Asn Ile Val Lys Pro Leu Pro
   225                 230                 235                 240
   Pro Val Tyr Leu Thr Phe Thr Arg Glu Ser Ser Cys Glu Ile Lys Leu
                   245                 250                 255
   Lys Trp Ser Ile Pro Leu Gly Pro Ile Pro Ala Arg Cys Phe Asp Tyr
               260                 265                 270
   Glu Ile Glu Ile Arg Glu Asp Asp Thr Thr Leu Val Thr Ala Thr Val
           275                 280                 285
   Glu Asn Glu Thr Tyr Thr Leu Lys Thr Thr Asn Glu Thr Arg Gln Leu
       290                 295                 300
   Cys Phe Val Val Arg Ser Lys Val Asn Ile Tyr Cys Ser Asp Asp Gly
   305                 310                 315                 320
   Ile Trp Ser Glu Trp Ser Asp Lys Gln Cys Trp Glu Gly Glu Asp Leu
                   325                 330                 335
   Ser Lys Lys Thr Leu Leu Arg Phe Trp Leu Pro Phe Gly Phe Ile Leu
               340                 345                 350
   Ile Leu Val Ile Phe Val Thr Gly Leu Leu Leu Arg Lys Pro Asn Thr
           355                 360                 365
   Tyr Pro Lys Met Ile Pro Glu Phe Phe Cys Asp Thr
       370                 375                 380
<210> 3
   <211> 2354
   <212> DNA
   <213> Homo Sapiens
<400> 3
   tttttttccc ctgctctccc aggggccaga caccaccgcc ccacccctca cgccccacct      60
   ccctggggga tcctttccgc cccagccctg aaagcgttaa ccctggagct ttctgcacac      120
   cccccgaccg ctcccgccca agcttcctaa aaaagaaagg tgcaaagttt ggtccaggat      180
   agaaaaatga ctgatcaaag gcaggcgata cttcctgttg ccgggacgct atatataacg      240
   tgatgagcgc acgggctgcg gagacgcacc ggagcgctcg cccagccgcc gcctccaagc      300
   ccctgaggtt tccggggacc acaatgaaca acttgctgtg ctgcgcgctc gtgtttctgg      360
   acatctccat taagtggacc acccaggaaa cgtttcctcc aaagtacctt cattatgacg      420
   aagaaacctc tcatcagctg ttgtgtgaca aatgtcctcc tggtacctac ctaaaacaac      480
   actgtacagc aaagtggaag accgtgtgcg ccccttgccc tgaccactac tacacagaca      540
   gctggcacac cagtgacgag tgtctatact gcagccccgt gtgcaaggag ctgcagtacg      600
   tcaagcagga gtgcaatcgc acccacaacc gcgtgtgcga atgcaaggaa gggcgctacc      660
   ttgagataga gttctgcttg aaacatagga gctgccctcc tggatttgga gtggtgcaag      720
   ctggaacccc agagcgaaat acagtttgca aaagatgtcc agatgggttc ttctcaaatg 780
   agacgtcatc taaagcaccc tgtagaaaac acacaaattg cagtgtcttt ggtctcctgc 840
   taactcagaa aggaaatgca acacacgaca acatatgttc cggaaacagt gaatcaactc 900
   aaaaatgtgg aatagatgtt accctgtgtg aggaggcatt cttcaggttt gctgttccta 960
   caaagtttac gcctaactgg cttagtgtct tggtagacaa tttgcctggc accaaagtaa 1020
   acgcagagag tgtagagagg ataaaacggc aacacagctc acaagaacag actttccagc 1080
   tgctgaagtt atggaaacat caaaacaaag accaagatat agtcaagaag atcatccaag 1140
   atattgacct ctgtgaaaac agcgtgcagc ggcacattgg acatgctaac ctcaccttcg 1200
   agcagcttcg tagcttgatg gaaagcttac cgggaaagaa agtgggagca gaagacattg 1260
   aaaaaacaat aaaggcatgc aaacccagtg accagatcct gaagctgctc agtttgtggc 1320
   gaataaaaaa tggcgaccaa gacaccttga agggcctaat gcacgcacta aagcactcaa 1380
   agacgtacca ctttcccaaa actgtcactc agagtctaaa gaagaccatc aggttccttc 1440
   acagcttcac aatgtacaaa ttgtatcaga agttattttt agaaatgata ggtaaccagg 1500
   tccaatcagt aaaaataagc tgcttataac tggaaatggc cattgagctg tttcctcaca 1560
   attggcgaga tcccatggat gagtaaactg tttctcaggc acttgaggct ttcagtgata 1620
   tctttctcat taccagtgac taattttgcc acagggtact aaaagaaact atgatgtgga 1680
   gaaaggacta acatctcctc caataaaccc caaatggtta atccaactgt cagatctgga 1740
   tcgttatcta ctgactatat tttcccttat tactgcttgc agtaattcaa ctggaaatta 1800
   aaaaaaaaaa actagactcc attgtgcctt actaaatatg ggaatgtcta acttaaatag 1860
   ctttgagatt tcagctatgc tagaggcttt tattagaaag ccatattttt ttctgtaaaa 1920
   gttactaata tatctgtaac actattacag tattgctatt tatattcatt cagatataag 1980
   atttgtacat attatcatcc tataaagaaa cggtatgact taattttaga aagaaaatta 2040
   tattctgttt attatgacaa atgaaagaga aaatatatat ttttaatgga aagtttgtag 2100
   catttttcta ataggtactg ccatattttt ctgtgtggag tatttttata attttatctg 2160
   tataagctgt aatatcattt tatagaaaat gcattattta gtcaattgtt taatgttgga 2220
   aaacatatga aatataaatt atctgaatat tagatgctct gagaaattga atgtacctta 2280
   tttaaaagat tttatggttt tataactata taaatgacat tattaaagtt ttcaaattat 2340
   tttttaaaaa aaaa 2354
<210> 4
   <211> 401
   <212> PRT
   <213> Homo Sapiens
<400> 4
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Leu Gln Tyr Val Lys Gln Glu
   85 90 95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
       290                 295                 300
   Lys Lys Val Gly Ala Glu Asp Ile Glu Lys Thr Ile Lys Ala Cys Lys
   305                 310                 315                 320
   Pro Ser Asp Gln Ile Leu Lys Leu Leu Ser Leu Trp Arg Ile Lys Asn
                   325                 330                 335
   Gly Asp Gln Asp Thr Leu Lys Gly Leu Met His Ala Leu Lys His Ser
               340                 345                 350
   Lys Thr Tyr His Phe Pro Lys Thr Val Thr Gln Ser Leu Lys Lys Thr
           355                 360                 365
   Ile Arg Phe Leu His Ser Phe Thr Met Tyr Lys Leu Tyr Gln Lys Leu
       370                 375                 380
   Phe Leu Glu Met Ile Gly Asn Gln Val Gln Ser Val Lys Ile Ser Cys
   385                 390                 395                 400
   Leu
<210> 5
   <211> 3897
   <212> DNA
   <213> Homo Sapiens
<400> 5
   cagtttgcaa aagccagagg tgcaagaagc agcgactgca gcagcagcag cagcagcggc 60
   ggtggcagca gcagcagcag cggcggcagc agcagcagca gcggaggcac cggtggcagc 120
   agcagcatca ccagcaacaa caacaaaaaa aaatcctcat caaatcctca cctaagcttt 180
   cagtgtatcc agatccacat cttcactcaa gccaggagag ggaaagagga aaggggggca 240
   ggaaaaaaaa aaaacccaac aacttagcgg aaacttctca gagaatgctc caaaactcag 300
   cagtgcttct ggtgctggtg atcagtgctt ctgcaaccca tgaggcggag cagaatgact 360
   ctgtgagccc caggaaatcc cgagtggcgg ctcaaaactc agctgaagtg gttcgttgcc 420
   tcaacagtgc tctacaggtc ggctgcgggg cttttgcatg cctggaaaac tccacctgtg 480
   acacagatgg gatgtatgac atctgtaaat ccttcttgta cagcgctgct aaatttgaca 540
   ctcagggaaa agcattcgtc aaagagagct taaaatgcat cgccaacggg gtcacctcca 600
   aggtcttcct cgccattcgg aggtgctcca ctttccaaag gatgattgct gaggtgcagg 660
   aagagtgcta cagcaagctg aatgtgtgca gcatcgccaa gcggaaccct gaagccatca 720
   ctgaggtcgt ccagctgccc aatcacttct ccaacagata ctataacaga cttgtccgaa 780
   gcctgctgga atgtgatgaa gacacagtca gcacaatcag agacagcctg atggagaaaa 840
   ttgggcctaa catggccagc ctcttccaca tcctgcagac agaccactgt gcccaaacac 900
   acccacgagc tgacttcaac aggagacgca ccaatgagcc gcagaagctg aaagtcctcc 960
   tcaggaacct ccgaggtgag gaggactctc cctcccacat caaacgcaca tcccatgaga 1020
   gtgcataacc agggagaggt tattcacaac ctcaccaaac tagtatcatt ttaggggtgt 1080
   tgacacacca gttttgagtg tactgtgcct ggtttgattt ttttaaagta gttcctattt 1140
   tctatccccc ttaaagaaaa ttgcatgaaa ctaggcttct gtaatcaata tcccaacatt 1200
   ctgcaatggc agcattccca ccaacaaaat ccatgtgacc attctgcctc tcctcaggag 1260
   aaagtaccct cttttaccaa cttcctctgc catgtttttc ccctgctccc ctgagaccac 1320
   ccccaaacac aaaacattca tgtaactctc cagccattgt aatttgaaga tgtggatccc 1380
   tttagaacgg ttgccccagt agagttagct gataaggaaa ctttatttaa atgcatgtct 1440
   taaatgctca taaagatgtt aaatggaatt cgtgttatga atctgtgctg gccatggacg 1500
   aatatgaatg tcacatttga attcttgatc tctaatgagc tagtgtctta tggtcttgat 1560
   cctccaatgt ctaattttct ttccgacaca tttaccaaat tgcttgagcc tggctgtcca 1620
   accagacttt gagcctgcat cttcttgcat ctaatgaaaa acaaaaagct aacatcttta 1680
   cgtactgtaa ctgctcagag ctttaaaagt atctttaaca attgtcttaa aaccagagaa 1740
   tcttaaggtc taactgtgga atataaatag ctgaaaacta atgtactgta cataaattcc 1800
   agaggactct gcttaaacaa agcagtatat aataacttta ttgcatatag atttagtttt 1860
   gtaacttagc tttatttttc ttttcctggg aatggaataa ctatctcact tccagatatc 1920
   cacataaatg ctccttgtgg ccttttttat aactaagggg gtagaagtag ttttaattca 1980
   acatcaaaac ttaagatggg cctgtatgag acaggaaaaa ccaacaggtt tatctgaagg 2040
   accccaggta agatgttaat ctcccagccc acctcaaccc agaggctact cttgacttag 2100
   acctatactg aaagatctct gtcacatcca actggaaatt ccaggaacca aaaagagcat 2160
   ccctatgggc ttggaccact tacagtgtga taaggcctac tatacattag gaagtggcag 2220
   ttctttactc gtcccctttc atcggtgcct ggtactctgg caaatgatga tggggtggga 2280
   gactttccat taaatcaatc aggaatgagt caatcagcct ttaggtcttt agtccggggg 2340
   acttggggct gagagagtat aaataaccct gggctgtcca gccttaatag acttctctta 2400
   cattttcgtc ctgtagcacg ctgcctgcca aagtagtcct ggcagctgga ccatctctgt 2460
   aggatcgtaa aaaaatagaa aaaaagaaaa aaaaaagaaa gaaagaggga aaaagagctg 2520
   gtggtttgat catttctgcc atgatgttta caagatggcg accaccaaag tcaaacgact 2580
   aacctatcta tgaacaacag tagtttctca gggtcactgt ccttgaaccc aacagtccct 2640
   tatgagcgtc actgcccacc aaaggtcaat gtcaagagag gaagagaggg aggaggggta 2700
   ggactgcagg ggccactcca aactcgctta ggtagaaact attggtgctt gactctcact 2760
   aggctaaact caagatttga ccaaatcgag tgatagggat cctggtggga ggagagaggg 2820
   cacatctcca gaaaaatgaa aagcaataca actttaccat aaagccttta aaaccagtaa 2880
   cgtgctgctc aaggaccaag agcaattgca gcagacccag cagcagcagc agcagcacaa 2940
   acattgctgc ctttgtcccc acacagcctc taagcgtgct gacatcagat tgttaagggc    3000
   atttttatac tcagaactgt cccatcccca ggtccccaaa cttatggaca ctgccttagc    3060
   ctcttggaaa tcaggtagac catattctaa gttagactct tcccctccct cccacacttc    3120
   ccacccccag gcaaggctga cttctctgaa tcagaaaagc tattaaagtt tgtgtgttgt     3180
   gtccattttg caaacccaac taagccagga ccccaatgcg acaagtagtt catgagtatt    3240
   cctagcaaat ttctctcttt cttcagttca gtagatttcc ttttttcttt tctttttttt    3300
   tttttttttt tttggctgtg acctcttcaa accgtggtac cccccctttt ctccccacga     3360
   tgatatctat atatgtatct acaatacata tatctacaca tacagaaaga agcagttctc    3420
   acaatgttgc tagttttttg cttctctttc ccccacccta ctccctccaa ttccccctta    3480
   aacttccaaa gcttcgtctt gtgtttgctg cagagtgatt cgggggctga cctagaccag    3540
   tttgcatgat tcttctcttg tgatttggtt gcactttaga catttttgtg ccattatatt    3600
   tgcattatgt atttataatt taaatgatat ttaggttttt ggctgagtac tggaataaac    3660
   agtgagcata tctggtatat gtcattattt attgttaaat tacattttta agctccatgt    3720
   gcatataaag gttatgaaac atatcatggt aatgacagat gcaagttatt ttatttgctt    3780
   atttttataa ttaaagatgc catagcataa tatgaagcct ttggtgaatt ccttctaaga    3840
   taaaaataat aataaagtgt tacgttttat tggtttcaaa aaaaaaaaaa aaaaaaa       3897
<210> 6
   <211> 247
   <212> PRT
   <213> Homo Sapiens
<400> 6
   Met Leu Gln Asn Ser Ala Val Leu Leu Val Leu Val Ile Ser Ala Ser
   1               5                   10                  15
   Ala Thr His Glu Ala Glu Gln Asn Asp Ser Val Ser Pro Arg Lys Ser
               20                  25                  30
   Arg Val Ala Ala Gln Asn Ser Ala Glu Val Val Arg Cys Leu Asn Ser
           35                  40                  45
   Ala Leu Gln Val Gly Cys Gly Ala Phe Ala Cys Leu Glu Asn Ser Thr
       50                  55                  60
   Cys Asp Thr Asp Gly Met Tyr Asp Ile Cys Lys Ser Phe Leu Tyr Ser
   65                  70                  75                  80
   Ala Ala Lys Phe Asp Thr Gln Gly Lys Ala Phe Val Lys Glu Ser Leu
                   85                  90                  95
   Lys Cys Ile Ala Asn Gly Val Thr Ser Lys Val Phe Leu Ala Ile Arg
               100                 105                 110
   Arg Cys Ser Thr Phe Gln Arg Met Ile Ala Glu Val Gln Glu Glu Cys
   115 120 125
   Tyr Ser Lys Leu Asn Val Cys Ser Ile Ala Lys Arg Asn Pro Glu Ala
   130 135 140
   Ile Thr Glu Val Val Gln Leu Pro Asn His Phe Ser Asn Arg Tyr Tyr
   145 150 155 160
   Asn Arg Leu Val Arg Ser Leu Leu Glu Cys Asp Glu Asp Thr Val Ser
   165 170 175
   Thr Ile Arg Asp Ser Leu Met Glu Lys Ile Gly Pro Asn Met Ala Ser
   180 185 190
   Leu Phe His Ile Leu Gln Thr Asp His Cys Ala Gln Thr His Pro Arg
   195 200 205
   Ala Asp Phe Asn Arg Arg Arg Thr Asn Glu Pro Gln Lys Leu Lys Val
   210 215 220
   Leu Leu Arg Asn Leu Arg Gly Glu Glu Asp Ser Pro Ser His Ile Lys
   225 230 235 240
   Arg Thr Ser His Glu Ser Ala
   245
<210> 7
   <211> 4507
   <212> DNA
   <213> Homo Sapiens
<400> 7
   gaccaattgt catacgactt gcagtgagcg tcaggagcac gtccaggaac tcctcagcag      60
   cgcctccttc agctccacag ccagacgccc tcagacagca aagcctaccc ccgcgccgcg     120
   ccctgcccgc cgctgcgatg ctcgcccgcg ccctgctgct gtgcgcggtc ctggcgctca     180
   gccatacagc aaatccttgc tgttcccacc catgtcaaaa ccgaggtgta tgtatgagtg     240
   tgggatttga ccagtataag tgcgattgta cccggacagg attctatgga gaaaactgct     300
   caacaccgga atttttgaca agaataaaat tatttctgaa acccactcca aacacagtgc     360
   actacatact tacccacttc aagggatttt ggaacgttgt gaataacatt cccttccttc     420
   gaaatgcaat tatgagttat gtgttgacat ccagatcaca tttgattgac agtccaccaa     480
   cttacaatgc tgactatggc tacaaaagct gggaagcctt ctctaacctc tcctattata     540
   ctagagccct tcctcctgtg cctgatgatt gcccgactcc cttgggtgtc aaaggtaaaa     600
   agcagcttcc tgattcaaat gagattgtgg aaaaattgct tctaagaaga aagttcatcc     660
   ctgatcccca gggctcaaac atgatgtttg cattctttgc ccagcacttc acgcatcagt     720
   ttttcaagac agatcataag cgagggccag ctttcaccaa cgggctgggc catggggtgg     780
   acttaaatca tatttacggt gaaactctgg ctagacagcg taaactgcgc cttttcaagg 840
   atggaaaaat gaaatatcag ataattgatg gagagatgta tcctcccaca gtcaaagata 900
   ctcaggcaga gatgatctac cctcctcaag tccctgagca tctacggttt gctgtggggc 960
   aggaggtctt tggtctggtg cctggtctga tgatgtatgc cacaatctgg ctgcgggaac 1020
   acaacagagt atgcgatgtg cttaaacagg agcatcctga atggggtgat gagcagttgt 1080
   tccagacaag caggctaata ctgataggag agactattaa gattgtgatt gaagattatg 1140
   tgcaacactt gagtggctat cacttcaaac tgaaatttga cccagaacta cttttcaaca 1200
   aacaattcca gtaccaaaat cgtattgctg ctgaatttaa caccctctat cactggcatc 1260
   cccttctgcc tgacaccttt caaattcatg accagaaata caactatcaa cagtttatct 1320
   acaacaactc tatattgctg gaacatggaa ttacccagtt tgttgaatca ttcaccaggc 1380
   aaattgctgg cagggttgct ggtggtagga atgttccacc cgcagtacag aaagtatcac 1440
   aggcttccat tgaccagagc aggcagatga aataccagtc ttttaatgag taccgcaaac 1500
   gctttatgct gaagccctat gaatcatttg aagaacttac aggagaaaag gaaatgtctg 1560
   cagagttgga agcactctat ggtgacatcg atgctgtgga gctgtatcct gcccttctgg 1620
   tagaaaagcc tcggccagat gccatctttg gtgaaaccat ggtagaagtt ggagcaccat 1680
   tctccttgaa aggacttatg ggtaatgtta tatgttctcc tgcctactgg aagccaagca 1740
   cttttggtgg agaagtgggt tttcaaatca tcaacactgc ctcaattcag tctctcatct 1800
   gcaataacgt gaagggctgt ccctttactt cattcagtgt tccagatcca gagctcatta 1860
   aaacagtcac catcaatgca agttcttccc gctccggact agatgatatc aatcccacag 1920
   tactactaaa agaacgttcg actgaactgt agaagtctaa tgatcatatt tatttattta 1980
   tatgaaccat gtctattaat ttaattattt aataatattt atattaaact ccttatgtta 2040
   cttaacatct tctgtaacag aagtcagtac tcctgttgcg gagaaaggag tcatacttgt 2100
   gaagactttt atgtcactac tctaaagatt ttgctgttgc tgttaagttt ggaaaacagt 2160
   ttttattctg ttttataaac cagagagaaa tgagttttga cgtcttttta cttgaatttc 2220
   aacttatatt ataagaacga aagtaaagat gtttgaatac ttaaacactg tcacaagatg 2280
   gcaaaatgct gaaagttttt acactgtcga tgtttccaat gcatcttcca tgatgcatta 2340
   gaagtaacta atgtttgaaa ttttaaagta cttttggtta tttttctgtc atcaaacaaa 2400
   aacaggtatc agtgcattat taaatgaata tttaaattag acattaccag taatttcatg 2460
   tctacttttt aaaatcagca atgaaacaat aatttgaaat ttctaaattc atagggtaga 2520
   atcacctgta aaagcttgtt tgatttctta aagttattaa acttgtacat ataccaaaaa 2580
   gaagctgtct tggatttaaa tctgtaaaat cagtagaaat tttactacaa ttgcttgtta 2640
   aaatatttta taagtgatgt tcctttttca ccaagagtat aaaccttttt agtgtgactg 2700
   ttaaaacttc cttttaaatc aaaatgccaa atttattaag gtggtggagc cactgcagtg 2760
   ttatcttaaa ataagaatat tttgttgaga tattccagaa tttgtttata tggctggtaa 2820
   catgtaaaat ctatatcagc aaaagggtct acctttaaaa taagcaataa caaagaagaa 2880
   aaccaaatta ttgttcaaat ttaggtttaa acttttgaag caaacttttt tttatccttg 2940
   tgcactgcag gcctggtact cagattttgc tatgaggtta atgaagtacc aagctgtgct 3000
   tgaataatga tatgttttct cagattttct gttgtacagt ttaatttagc agtccatatc 3060
   acattgcaaa agtagcaatg acctcataaa atacctcttc aaaatgctta aattcatttc 3120
   acacattaat tttatctcag tcttgaagcc aattcagtag gtgcattgga atcaagcctg 3180
   gctacctgca tgctgttcct tttcttttct tcttttagcc attttgctaa gagacacagt 3240
   cttctcatca cttcgtttct cctattttgt tttactagtt ttaagatcag agttcacttt 3300
   ctttggactc tgcctatatt ttcttacctg aacttttgca agttttcagg taaacctcag 3360
   ctcaggactg ctatttagct cctcttaaga agattaaaag agaaaaaaaa aggccctttt 3420
   aaaaatagta tacacttatt ttaagtgaaa agcagagaat tttatttata gctaatttta 3480
   gctatctgta accaagatgg atgcaaagag gctagtgcct cagagagaac tgtacggggt 3540
   ttgtgactgg aaaaagttac gttcccattc taattaatgc cctttcttat ttaaaaacaa 3600
   aaccaaatga tatctaagta gttctcagca ataataataa tgacgataat acttcttttc 3660
   cacatctcat tgtcactgac atttaatggt actgtatatt acttaattta ttgaagatta 3720
   ttatttatgt cttattagga cactatggtt ataaactgtg tttaagccta caatcattga 3780
   tttttttttg ttatgtcaca atcagtatat cttctttggg gttacctctc tgaatattat 3840
   gtaaacaatc caaagaaatg attgtattaa gatttgtgaa taaattttta gaaatctgat 3900
   tggcatattg agatatttaa ggttgaatgt ttgtccttag gataggccta tgtgctagcc 3960
   cacaaagaat attgtctcat tagcctgaat gtgccataag actgaccttt taaaatgttt 4020
   tgagggatct gtggatgctt cgttaatttg ttcagccaca atttattgag aaaatattct 4080
   gtgtcaagca ctgtgggttt taatattttt aaatcaaacg ctgattacag ataatagtat 4140
   ttatataaat aattgaaaaa aattttcttt tgggaagagg gagaaaatga aataaatatc 4200
   attaaagata actcaggaga atcttcttta caattttacg tttagaatgt ttaaggttaa 4260
   gaaagaaata gtcaatatgc ttgtataaaa cactgttcac tgtttttttt aaaaaaaaaa 4320
   cttgatttgt tattaacatt gatctgctga caaaacctgg gaatttgggt tgtgtatgcg 4380
   aatgtttcag tgcctcagac aaatgtgtat ttaacttatg taaaagataa gtctggaaat 4440
   aaatgtctgt ttatttttgt actatttaaa aattgacaga tcttttctga agaaaaaaaa 4500
   aaaaaaa 4507
<210> 8
   <211> 604
   <212> PRT
   <213> Homo Sapiens
<400> 8
<210> 9
   <211> 2354
   <212> DNA
   <213> Homo Sapiens
<400> 9
   gctcagggca catgcctccc ctccccaggc cgcggcccag ctgaccctcg gggctccccc 60
   ggcagcggac agggaagggt taaaggcccc cggctccctg ccccctgccc tggggaaccc 120
   ctggccctgt ggggacatga actgtgtttg ccgcctggtc ctggtcgtgc tgagcctgtg 180
   gccagataca gctgtcgccc ctgggccacc acctggcccc cctcgagttt ccccagaccc 240
   tcgggccgag ctggacagca ccgtgctcct gacccgctct ctcctggcgg acacgcggca 300
   gctggctgca cagctgaggg acaaattccc agctgacggg gaccacaacc tggattccct 360
   gcccaccctg gccatgagtg cgggggcact gggagctcta cagctcccag gtgtgctgac 420
   aaggctgcga gcggacctac tgtcctacct gcggcacgtg cagtggctgc gccgggcagg 480
   tggctcttcc ctgaagaccc tggagcccga gctgggcacc ctgcaggccc gactggaccg 540
   gctgctgcgc cggctgcagc tcctgatgtc ccgcctggcc ctgccccagc cacccccgga 600
   cccgccggcg cccccgctgg cgcccccctc ctcagcctgg gggggcatca gggccgccca 660
   cgccatcctg ggggggctgc acctgacact tgactgggcc gtgaggggac tgctgctgct 720
   gaagactcgg ctgtgacccg gggcccaaag ccaccaccgt ccttccaaag ccagatctta 780
   tttatttatt tatttcagta ctgggggcga aacagccagg tgatcccccc gccattatct 840
   ccccctagtt agagacagtc cttccgtgag gcctgggggg catctgtgcc ttatttatac 900
   ttatttattt caggagcagg ggtgggaggc aggtggactc ctgggtcccc gaggaggagg 960
   ggactggggt cccggattct tgggtctcca agaagtctgt ccacagactt ctgccctggc 1020
   tcttccccat ctaggcctgg gcaggaacat atattattta tttaagcaat tacttttcat 1080
   gttggggtgg ggacggaggg gaaagggaag cctgggtttt tgtacaaaaa tgtgagaaac 1140
   ctttgtgaga cagagaacag ggaattaaat gtgtcataca tatccacttg agggcgattt 1200
   gtctgagagc tggggctgga tgcttgggta actggggcag ggcaggtgga ggggagacct 1260
   ccattcaggt ggaggtcccg agtgggcggg gcagcgactg ggagatgggt cggtcaccca 1320
   gacagctctg tggaggcagg gtctgagcct tgcctggggc cccgcactgc atagggcctt 1380
   ttgtttgttt tttgagatgg agtctcgctc tgttgcctag gctggagtgc agtgaggcaa 1440
   tctgaggtca ctgcaacctc cacctcccgg gttcaagcaa ttctcctgcc tcagcctccc   1500
   gattagctgg gatcacaggt gtgcaccacc atgcccagct aattatttat ttcttttgta   1560
   tttttagtag agacagggtt tcaccatgtt ggccaggctg gtttcgaact cctgacctca   1620
   ggtgatcctc ctgcctcggc ctcccaaagt gctgggatta caggtgtgag ccaccacacc   1680
   tgacccatag gtcttcaata aatatttaat ggaaggttcc acaagtcacc ctgtgatcaa   1740
   cagtacccgt atgggacaaa gctgcaaggt caagatggtt cattatggct gtgttcacca   1800
   tagcaaactg gaaacaatct agatatccaa cagtgagggt taagcaacat ggtgcatctg   1860
   tggatagaac gccacccagc cgcccggagc agggactgtc attcagggag gctaaggaga   1920
   gaggcttgct tgggatatag aaagatatcc tgacattggc caggcatggt ggctcacgcc   1980
   tgtaatcctg gcactttggg aggacgaagc gagtggatca ctgaagtcca agagttcgag   2040
   accggcctgc gagacatggc aaaaccctgt ctcaaaaaag aaagaatgat gtcctgacat   2100
   gaaacagcag gctacaaaac cactgcatgc tgtgatccca attttgtgtt tttctttcta   2160
   tatatggatt aaaacaaaaa tcctaaaggg aaatacgcca aaatgttgac aatgactgtc   2220
   tccaggtcaa aggagagagg tgggattgtg ggtgactttt aatgtgtatg attgtctgta   2280
   ttttacagaa tttctgccat gactgtgtat tttgcatgac acattttaaa aataataaac   2340
   actattttta gaat                                                     2354
<210> 10
   <211> 199
   <212> PRT
   <213> Homo Sapiens
<400> 10
   Met Asn Cys Val Cys Arg Leu Val Leu Val Val Leu Ser Leu Trp Pro
   1               5                   10                  15
   Asp Thr Ala Val Ala Pro Gly Pro Pro Pro Gly Pro Pro Arg Val Ser
               20                  25                  30
   Pro Asp Pro Arg Ala Glu Leu Asp Ser Thr Val Leu Leu Thr Arg Ser
           35                  40                  45
   Leu Leu Ala Asp Thr Arg Gln Leu Ala Ala Gln Leu Arg Asp Lys Phe
       50                  55                  60
   Pro Ala Asp Gly Asp His Asn Leu Asp Ser Leu Pro Thr Leu Ala Met
   65                  70                  75                  80
   Ser Ala Gly Ala Leu Gly Ala Leu Gln Leu Pro Gly Val Leu Thr Arg
                   85                  90                  95
   Leu Arg Ala Asp Leu Leu Ser Tyr Leu Arg His Val Gln Trp Leu Arg
               100                 105                 110
   Arg Ala Gly Gly Ser Ser Leu Lys Thr Leu Glu Pro Glu Leu Gly Thr
           115                 120                 125
   Leu Gln Ala Arg Leu Asp Arg Leu Leu Arg Arg Leu Gln Leu Leu Met
       130                 135                 140
   Ser Arg Leu Ala Leu Pro Gln Pro Pro Pro Asp Pro Pro Ala Pro Pro
   145                 150                 155                 160
   Leu Ala Pro Pro Ser Ser Ala Trp Gly Gly Ile Arg Ala Ala His Ala
                   165                 170                 175
   Ile Leu Gly Gly Leu His Leu Thr Leu Asp Trp Ala Val Arg Gly Leu
               180                 185                 190
   Leu Leu Leu Lys Thr Arg Leu
           195